# EUROPÄISCHE PATENTANMELDUNG

(11) **EP 4 715 054 A1**
(43) Veröffentlichungstag der Anmeldung: **25.03.2026**
(21) Anmeldenummer: 25199297.0
(22) Anmeldetag: 01.09.2025
(51) Int. Cl.: C12N 15/115

(54) **APTAMERE, APTAMERBASIERTE SENSOREN UND VERFAHREN FÜR DEN NACHWEIS VON NEUROFILAMENT LIGHT**

(30) Priorität: 02.09.2024 DE 102024124992
(71) Anmelder: Forschungszentrum Jülich GmbH, 52428 Jülich (DE)
(72) Erfinder: MAYER, Dirk, 50226 Frechen (DE); Li, Hangyu, 52428 Jülich (DE); PERCZE, Krisztina, 1148 Budapest (HU); OFFENHÄUSER, Andreas, 52066 Aachen (DE)
(74) Vertreter: Meissner Bolte Düsseldorf Patentanwälte Rechtsanwälte Partnerschaft mbB

(57) **Zusammenfassung**

Die vorliegende Erfindung betrifft Aptamere, die an Neurofilament Light binden, Biosensoren, Verwendungen der Aptamere, Verfahren zum Nachweis von Neurofilament Light.

## Beschreibung

Die vorliegende Erfindung betrifft Aptamermoleküle, die selektiv und mit hoher Affinität an Neurofilament Light binden, Sensoren für den Nachweis von Neurofilament Light, Verfahren zur Bestimmung von Neurofilament Light, sowie entsprechend Anund Verwendungen.

### Stand der Technik:

Gegenwärtig erfolgt die Bestimmung von Biomarkern für die Diagnostik von Demenz wie Alzheimererkrankung mit Hilfe von Antikörpern. Die Biomarker werden vorrangig in Cerebrospinalflüssigkeit (CSF) bestimmt, was durch eine erforderliche Lumbalpunktion invasiv für den Patienten ist. Die Tests erfolgen in der Regel mit Enzyme-linked Immunosorbent Assay (ELISA). In einigen Fällen wird auch Positronen-Emissions-Tomographie (PET) zum Nachweis von Biomarkern in betroffenem Gewebe verwendet. PET-Untersuchungen sind sehr teuer und ebenfalls invasiv. Diese Methoden werden verwendet, wenn schon eine Indikation für die Erkrankung vorliegt und nicht für Screenings ohne vorherige symptomatische Indikationen. Zudem sind ELISA Tests teuer, benötigen lange Testzeiten, und erfahrenes Personal. Die verwendeten Antikörper sind empfindlich hinsichtlich ihrer Degradation, weisen Variationen in der Bindungscharakteristik auf und ihre Entwicklung erfordert Tierexperimente. Die gegenwärtigen Biomarker-Tests werden aufgrund deren hoher Preise und Invasivität in der Regel erst durchgeführt, wenn schon kognitive Beeinträchtigungen bei den Patienten festgestellt wurden, um die Diagnose zu bestätigen. Je später die Diagnose erfolgt, desto weniger Interventionsmöglichkeiten bestehen und deren Erfolgsaussichten nehmen ab. Typischerweise beginnen die molekularen Prozesse der Neurodegeneration, die zur Alzheimererkrankung führen, Jahre bevor kognitive Beeinträchtigungen auftreten.

Ein Biomarker für die Indikation von Demenzerkrankungen, insbesondere von Alzheimererkrankungen, ist Neurofilament Light (NF-L oder NFL). Neurofilament Light (NFL) ist ein Protein, das in den axonalen Neuriten von Neuronen vorkommt und infolge von neuronalen Degradationen oder Verletzungen in die interstitielle Gewebeflüssigkeit vordringt. NFL-Konzentrationen sind für Patienten mit neurodegenerativen Erkrankungen auch schon in der vorsymptomatischen Phase ohne quantifizierbare kognitive Einschränkungen erhöht. Daher kann der Nachweis von erhöhten Werten von NFL in Körperflüssigkeiten die Diagnose von neurodegenerativen Erkrankungen in frühen Stadien unterstützen, in denen eine Behandlung höhere Erfolgschancen hat.

Als Stand der Technik können unter anderem US 2023/0266343 A1 und US 2023/0190967 A1 genannt werden.

### Aufgabe:

Aufgabe der vorliegenden Erfindung war es, Verfahren und Messvorrichtungen für die Bestimmung von Biomarkern für Alzheimer, insbesondere Neurofilament Light (NFL), in Proben, bevorzugt Körperflüssigkeiten und/oder Stuhl, besonders bevorzugt Speichel, Urin, Serum, Blut und Cerebrospinalflüssigkeit (CSF), insbesondere Blut, bereitgestellt werden, um die Diagnose von Alzheimer Erkrankungen (AD) zu unterstützen. Die Bestimmung sollte möglichst selektiv und mit möglichst hoher Affinität möglich sein.

Außerdem sollen die bereitzustellenden Verfahren und Messvorrichtungen am Point-of-Care verwendbar sein, kostengünstig herzustellen sein, eine hohe Stabilität aufweisen, einfach zu bedienen sein und eine hohe Testgenauigkeit aufweisen.

Weitere Aufgabenstellungen ergeben sich für den Fachmann bei Betrachtung der nachfolgenden Beschreibung und der Ansprüche.

### Lösung:

Diese und weitere Aufgaben, die sich dem Fachmann bei Betrachtung der vorliegenden Beschreibung erschließen, werden durch die in den unabhängigen Ansprüchen dargestellten Gegenstände gelöst.

Besonders vorteilhafte und bevorzugte Gegenstände ergeben sich aus den abhängigen Ansprüchen sowie der nachfolgenden Beschreibung.

Bevorzugte Ausgestaltungen ergeben sich aus den abhängigen Ansprüchen und/oder der folgenden Beschreibung.

### Detaillierte Beschreibung:

Im Rahmen der vorliegenden Erfindung bedeutet Raumtemperatur eine Temperatur von 293,15 Kelvin, d.h. 20°C.

Soweit nicht weiter ausgeführt herrscht bei allen Reaktionen und Vorgehensweisen Atmosphärendruck (1013 mbar_{absolut}) und Raumtemperatur.

Im Rahmen der vorliegenden Erfindung werden die Begriffe "Sensor" und "Biosensor" synonym verwendet.

Im Rahmen der vorliegenden Erfindung sind Nanoobjekte bzw. Nanopartikel Objekte mit mindestens einer Raumdimension kleiner 100 nm, gemessen per Rasterelektronenmikroskopie.

Im Rahmen der vorliegenden Erfindung umfasst der Begriff "Neurofilament Light" "Neurofilament Light chain", "Neurofilament Light Polypeptid" und "Neurofilament Light".

Die vorliegende Erfindung betrifft in einem ersten Gegenstand insbesondere Aptamere, insbesondere DNA-Aptamere, die an Neurofilament Light binden und ausgewählt sind aus der Gruppe bestehend aus
a) einem Aptamer umfassend, oder bestehend aus, eine/einer Nukleinsäuresequenz, die ausgewählt ist aus der Gruppe bestehend aus SEQ ID NO: 1, 2 und 3,
b) einem Aptamer, dessen Nukleinsäuresequenz eine Übereinstimmung von mindestens 50%, 60%, 65%, 70%, 75%, 80%, 85%, 90%, 95% oder 98% mit der Nukleinsäuresequenz eines Aptamers aus a) aufweist,
c) einem Aptamer, bei dem gegenüber einem Aptamer aus a) bei der Sequenz mit der SEQ ID NO: 1, 2 oder 3 bis zu 19 Nukleotide, insbesondere 1, 2, 3, 4, 5, 6, 7, 8, 9, 10, 11, 12, 13, 14, 15, 16, 17, 18 oder 19 Nukleotide, entfernt, substituiert und/oder erweitert sind.

Der allgemeine Begriff "Aptamere" bezeichnet im Stand der Technik kurze einzelsträngige Nukleinsäure-Oligomere, auch bezeichnet als Oligonukleotide, die spezifisch an eine Zielstruktur oder ein Zielmolekül, auch bezeichnet als Target, binden können, beispielsweise an ein Protein, an niedermolekulare Verbindungen, wie organische Substanzen, Aminosäuren und Antibiotika, Nukleinsäuren, Viruspartikel oder (Mikro)Organismen. Die Aptamer-Target-Bindung erfolgt beispielsweise über die Strukturkompatibilität, so genannte "Stacking interactions" bei aromatischen Ringstrukturen (Stapelkräfte durch Elektronenwechselwirkung mit Nachbarbasen), elektrostatische Wechselwirkungen (z.B. Van der Waals-, Ionen-, Dipolkräfte) und Wasserstoffbrückenbindungen.

Auch Aptamere mit Peptidstruktur sind bekannt, wobei sich die vorliegende Erfindung ausschließlich auf Nukleinsäure-Aptamere bezieht. Somit bezeichnet der Begriff "Aptamere" nachfolgend Nukleinsäure-Aptamere. Bei Nukleinsäure-Aptameren unterscheidet man beispielsweise DNA-Aptamere, gebildet aus einzelsträngiger DNA (ssDNA), und RNA-Aptamere. Aptamere zeichnen sich durch die Ausbildung einer spezifischen dreidimensionalen Struktur, die von der Nukleinsäuresequenz abhängt, aus. Diese Struktur befähigt Aptamere, analog einer Antigen-Antikörper-Bindung Zielstrukturen passgenau zu binden. Eine bestimmte Nukleinsäuresequenz eines Aptamers kann bei definierten Bedingungen eine dreidimensionale Struktur aufweisen, die spezifisch für eine definierte Zielstruktur (Target) ist. Die dreidimensionale Struktur eines Aptamers entsteht u.a. infolge von intramolekularen Basenpaarungen nach Watson und Crick und über Hoogsteen-Basenpaarungen (Quadruplex).

In bevorzugten Ausgestaltungen der vorliegenden Erfindung sind die Aptamere der vorliegenden Erfindung durch Bindung, insbesondere kovalente Bindung, an ein Sondenmolekül, bevorzugt ausgewählt aus der Gruppe bestehend aus Fluorophoren, Nanomaterialien, Enzymen, Redoxmolekülen oder Mischungen oder Kombinationen davon, insbesondere ein Fluorophor, insbesondere an dem 3'-Ende, modifiziert, um eine Signalwandlung zu ermöglichen oder zu verstärken.

In alternativen bevorzugten Ausgestaltungen der vorliegenden Erfindung sind die Aptamere der vorliegenden Erfindung durch Bindung, insbesondere kovalente Bindung, an ein Sondenmolekül, bevorzugt ausgewählt aus der Gruppe bestehend aus Fluorophoren, Nanomaterialien, Enzymen, Redoxmolekülen oder Mischungen oder Kombinationen davon, insbesondere ein Fluorophor, insbesondere an dem 3'-Ende, modifiziert, um eine Signalwandlung zu ermöglichen oder zu verstärken, und zusätzlich an seinem anderen Ende durch Bindung, insbesondere kovalente Bindung, an eine Bindungsgruppe modifiziert.

Ein weiterer Gegenstand der vorliegenden Erfindung betrifft Aptamersonden zur Detektion von Neurofilament Light umfassend ein oder mehrere verschiedene erfindungsgemäße Aptamere, wie oben oder weiter unten beschrieben, und ein Markierungsmittel, bevorzugt ausgewählt aus der Gruppe bestehend aus Lumineszenz-, Phosphoreszenz, Fluoreszenz-, radioaktiven, oder UV/VIS-Markierungssubstanzen, die, insbesondere als Reporter-, Marker- oder Adaptermoleküle, an die Aptamere binden/gebunden werden.

Noch ein weiterer Gegenstand der vorliegenden Erfindung sind Biosensoren umfassend ein oder mehrere verschiedene erfindungsgemäße Aptamere, wie oben oder weiter unten beschrieben oder erfindungsgemäße Aptamersonden.

Ebenfalls ein weiterer Gegenstand der vorliegenden Erfindung sind Biosensoren beruhend auf elektrochemischen, thermischen, optischen, elektrischen, magnetischen, gravimetrischen Prinzipien oder anderen Transducer-Prinzipien, insbesondere elektrochemischen, optischen oder elektrischen Prinzipien, insbesondere zur Bestimmung von Neurofilament Light in Körperflüssigkeiten und/oder Stuhl, bevorzugt Speichel, Urin, Blut, Serum oder CSF, insbesondere Blut, und umfassend
- ein an Neurofilament Light bindendes Aptamer oder mehrere solche Aptamere, bevorzugt beruhend auf einzelsträngiger Desoxyribonukleinsäure, besonders bevorzugt ein oder mehrere verschiedene erfindungsgemäße Aptamere, wie oben oder weiter unten beschrieben,
wobei die Aptamere entweder
a) frei in einer Probenlösung vorliegen, oder
b) an einer festen Phase, bevorzugt auf einer Elektrode, einer Transistorelektrode, insbesondere Gate-Elektrode oder dem Transistorkanal, einer Mikrowaage wie Schwingkristalle und Federarme, para- oder ferromagnetischen Materialien, optischen Bauelementen wie beschichteten und unbeschichteten Prismen, Spiegeln, Gitter, Faseroptiken, Polarisatoren u.a. , transparenten Substraten, Nanomaterialien, mikrooder nanostrukturierte Materialien, metallorganische Gerüstverbindungen (*metalorganic frameworks,* MOFs) oder einem anderem Signalwandler, immobilisiert vorliegen.

In bevorzugten Ausgestaltungen der Biosensoren der vorliegenden Erfindung ist das eingesetzte Aptamer durch Bindung, insbesondere kovalente Bindung, an ein Sondenmolekül, bevorzugt ausgewählt aus der Gruppe bestehend aus Fluorophoren, Nanomaterialien, Enzymen, Redoxmolekülen oder Mischungen oder Kombinationen davon, insbesondere ein Fluorophor, insbesondere an dem 3'-Ende, modifiziert ist, um eine Signalwandlung zu ermöglichen oder zu verstärken.

In alternativen bevorzugten Ausgestaltungen der Biosensoren der vorliegenden Erfindung ist das eingesetzte Aptamer durch Bindung, insbesondere kovalente Bindung, an ein Sondenmolekül, bevorzugt ausgewählt aus der Gruppe bestehend aus Fluorophoren, Nanomaterialien, Enzymen, Redoxmolekülen oder Kombinationen davon, insbesondere ein Fluorophor, insbesondere an dem 3'-Ende, modifiziert, um eine Signalwandlung zu ermöglichen oder zu verstärken. Zusätzlich oder alternativ kann das eingesetzte Aptamer an seinem anderen Ende durch Bindung, insbesondere kovalente Bindung, an eine Bindungsgruppe modifiziert werden.

Hierbei ist es in Ausführungsformen der vorliegenden Erfindung bevorzugt, wenn zusätzlich Moleküle, welche die unspezifische Bindung von Komponenten der Probe unterdrücken, an der festen Phase immobilisiert sind.

Ein weiterer Gegenstand der vorliegenden Erfindung ist eine feste Phase, bevorzugt Elektrode, Transistorelektrode, insbesondere Gate-Elektrode, Mikrowaage, oder ein anderer Signalwandler an bzw. auf der ein oder mehrere Aptamere oder modifizierte Aptamere der vorliegenden Erfindung wie weiter oben oder unten beschrieben immobilisiert sind, bevorzugt zum Nachweis, der Anreicherung, der Abtrennung oder der Isolierung von Neurofilament Light, insbesondere dem Nachweis von Neurofilament Light.

Ein weiterer Gegenstand der vorliegenden Erfindung sind Teststreifen, zum Beispiel zur Verwendung in Lateralfluss-Assays, umfassend ein oder mehrere Aptamere oder modifizierte Aptamere der vorliegenden Erfindung wie weiter oben oder unten beschrieben.

Ein weiterer Gegenstand der vorliegenden Erfindung ist ein Kit umfassend ein oder mehrere Aptamere oder modifizierte Aptamere der vorliegenden Erfindung wie weiter oben oder unten beschrieben und optional weiteres Analysenmaterial, bevorzugt Pipetten, Biosensorchips, transportable Lesegeräte, Chemikalien, Präparationsröhrchen (Eppendorf-Tubes), Küvetten, Lichtquelle, Fluoreszenzdetektor, und ähnliches. Im Zusammenhang mit dem Begriff "Kit" sei drauf hingewiesen, dass dieses Kit nicht zwangsweise für die "Hosentasche" geeignet sein muss.

Noch ein Gegenstand der vorliegenden Erfindung ist ein Verfahren zur Bestimmung von Neurofilament Light in Körperflüssigkeiten und/oder Stuhl, bevorzugt Speichel, Urin, Blut, Serum oder CSF, insbesondere Blut, umfassend die Schritte:
a) Bereitstellen eines an Neurofilament Light bindenden Aptamers oder mehrere solche Aptamere, bevorzugt beruhend auf einzelsträngiger Desoxyribonukleinsäure, besonders bevorzugt ein oder mehrere Aptamere oder modifizierte Aptamere der vorliegenden Erfindung wie weiter oben oder unten beschrieben;
b) Inkontaktbringen des Aptamers oder der Aptamere mit einer Probe der Körperflüssigkeit und/oder des Stuhls, bevorzugt Speichel, Urin, Blut, Serum oder CSF, insbesondere Blut;
c) Detektieren der Bindung von Neurofilament Light an das Aptamer oder die Aptamere.

In bevorzugten Ausgestaltungen des erfindungsgemäßen Verfahrens wird das Aptamer in Form eines erfindungsgemäßen Biosensors, wie oben oder weiter unten beschreiben, bereitgestellt.

In weiteren bevorzugten Ausgestaltungen des erfindungsgemäßen Verfahrens erfolgt die Detektion mittels
- optischer Methoden, bevorzugt durch Detektion von Fluoreszenzlicht, oder kolorimetrisch durch Farbänderung z.B. in Lateral Flow Assays oder
- elektrischer Methoden, bevorzugt Messung von Spannungsänderungen mit Hilfe eines Transistors, insbesondere eines OECT (organic electrochemical transistor), oder
- elektrochemischer Methoden, bevorzugt durch Messung von Probenströmen bzw. Widerstände mit Hilfe von Elektroden, insbesondere elektrochemischer Impedanzspektroskopie.

Das erfindungsgemäße Verfahren kann zum Beispiel auch in Form eines kompetitiven Assays oder eines Sandwich-Assays durchgeführt werden.

Ein weiterer erfindungsgemäßer Gegenstand ist ein Verfahren zur Herstellung der erfindungsgemäßen Biosensoren, wie oben oder weiter unten beschrieben, umfassend die Schritte
- Bereitstellen eines an Neurofilament Light bindenden Aptamers oder mehrerer solcher Aptamere, bevorzugt beruhend auf einzelsträngiger Desoxyribonukleinsäure, besonders bevorzugt ein oder mehrere Aptamere oder modifizierte Aptamere der vorliegenden Erfindung wie weiter oben oder unten beschrieben;
- optional, und in vielen Ausführungsformen der vorliegenden Erfindung bevorzugt, Modifizieren des wie oben oder weiter unten beschriebenen Aptamers oder der Aptamere;
- Bereitstellen einer Elektrode, einer Transistorelektrode, insbesondere Gate-Elektrode, einer Mikrowaage, oder einem anderem Signalwandler;
- Immobilisieren des Aptamers auf der Elektrode, der Mikrowaage oder dem anderen Signalwandler, bevorzugt über Bindungsgruppen, besonders bevorzugt über Thiolgruppen enthaltende Gruppe, insbesondere eine HS-(CH₂)₆-Gruppe;
- optional, und in vielen Ausführungsformen der vorliegenden Erfindung bevorzugt, Immobilisieren von Blockierungsmolekülen auf der der Elektrode, der Mikrowaage oder dem anderen Signalwandler.

Überdies ein weiterer Gegenstand der vorliegenden Erfindung ist die Verwendung der erfindungsgemäßen Aptamere wie oben oder weiter unten beschrieben, der erfindungsgemäßen Aptamersonden, der erfindungsgemäßen Biosensoren wie oben oder weiter unten beschrieben, des ersten erfindungsgemäßen Verfahrens wie oben oder weiter unten beschrieben,
- zum Nachweis von Neurofilament Light in Proben, bevorzugt in Körperflüssigkeiten und/oder Stuhl, bevorzugt Speichel, Urin, Blut, Serum oder CSF, insbesondere Blut;
- zur Anreicherung, Abtrennung und/oder Isolierung von Neurofilament Light aus Proben;
- für potentiell präventive und dezentrale Testung, insbesondere breiter Bevölkerungsgruppen.

Schließlich ist Gegenstand der vorliegenden Erfindung die Verwendung der erfindungsgemäßen Aptamere oder der Aptamersonden wie oben oder weiter unten beschrieben als Therapeutika, als Affinitätstags, als Reagenzien, zum Markieren von Systemen, die Neurofilament Light enthalten, für oder in Biosensoren, zur Qualitätskontrolle.

Bei der Verwendung der Aptamere der vorliegenden Erfindung als Therapeutika werden diese bevorzugt in der gezielten Therapie neurodegenerativer Erkrankungen verwendet. Die therapeutischen Effekte werden dabei zum Beispiel erzielt durch Neutralisation, gezielte Wirkstoffabgabe oder Immunmodulation, insbesondere, aber nicht ausschließlich, wie folgt:
1. Die erfindungsgemäßen Aptamere können freigesetztes NFL im Blut oder Liquor binden und neutralisieren, um zu verhindern, dass das freigesetzte NFL entzündliche oder toxische Prozesse fördert. Dadurch können auch sekundäre Schäden verhindert werden.
2. Die erfindungsgemäßen Aptamere können als Trägersysteme dienen, um Medikamente gezielt in Regionen mit hoher NFL-Konzentration zu bringen - also dort, wo neuronale Schädigung aktiv ist.
3. Die erfindungsgemäßen Aptamere können helfen, unerwünschte Immunreaktionen direkt an den Neuronen zu dämpfen, um immunologische Aktivität von NFL zu unterdrücken.

Die bei den erfindungsgemäßen Biosensoren eingesetzten Elektroden können auf Gold oder anderen Metallen, insbesondere Edelmetallen (unter anderem, weil diese ideal polarisierbar sind), Halbleitern, Kohlenstoff oder leitfähigen Polymeren beruhen. Besonders bevorzugtes Elektrodenmaterial im Rahmen der vorliegenden Erfindung ist Gold. Zudem können die Elektroden in weiteren bevorzugten Ausführungsformen Beschichtungen aufweisen, bevorzugt aus Nanomaterialien, Polymerfilmen oder Molekülen, die die Oberfläche der Elektroden funktionalisieren z.B. die Anzahl an Bindungsstellen erhöhen (Nanomaterialien z.B. nanoporöses Gold, Platinblack, nanostrukturiertes Gold, MOFs, dendritische Moleküle, polyvalente Moleküle) die Impedanz reduzieren (Nanomaterialien z.B. nanoporöses Gold, Platinblack, nanostrukturiertes Gold, leitfähige Polymere z.B. Poly-3,4-ethylendioxythiophen/Polystyrolsulfonsäure (PEDOT/PSS), die chemischen Eigenschaften der Elektroden verändern (hydrophobe Moleküle z.B. Alkanthiole, proteinaversive Moleküle z.B. Ethylenglykole, hydrophile Moleküle z.B. Polykationen oder Polyanionen, monomer-, oligomer- oder polymerartige Zucker) oder anderes.

Falls statt einfacher Elektroden Transistoren eingesetzt werden, wird im Rahmen der vorliegenden Erfindung bevorzugt eine Konfiguration verwendet, bei der Gate-Elektrode und Transistorkanal über eine Flüssigkeit miteinander verbunden sind (auch als Liquid Gate bezeichnet). Bei diesen kann der Kanal aus halbleitenden Polymerschichten (z.B. Poly(3-hexylthiophen) - P3HT, Polyparaphenylen, Polypyrrol, Polyanilin), aus Halbleitern (Si, Ge, GaAs und andere), aus Kohlenstoff (Graphen, reduziertes Graphenoxid, Kohlenstoffnanoröhren, Fulleren und anderen), aus Molekülen mit konjugiertem pi-System (Perylen, 3,4,9,10-Perylentetracarbonsäuredianhydrid - PTCDA und dessen Derivate, NaphthalinDerivate, Hexabenzocoronen und andere) oder aus leitfähigen Biomolekülen wie Proteinen (native und rekombinante Proteine z.B. von Bakterien wie Kabelbakterien) bestehen. Die Gate-Elektrode, dies ist die Elektrode, auf die die Aptamere aufgebracht werden, kann sich neben dem Kanal, auf dem Kanal oder in der Probenflüssigkeit befinden. Weiterhin kann eine Extended-Gate-Konfiguration zum Einsatz kommen, bei der sich das Gate in der Probenflüssigkeit befindet, der Kanal aber nicht. Im Rahmen der vorliegenden Erfindung werden bevorzugt OECT (organic electrochemical transistor) verwendet, deren Kanalmaterial aus leitfähigen Polymeren besteht, wobei besonders bevorzugt PEDOT/PSS als Kanalmaterial verwendet wird.-Die Aptamere können aber auch auf dem Kanalmaterial aufgebracht oder darin inkorporiert werden.

Die Elektroden der erfindungsgemäßen Biosensoren können in ihren Dimensionen und Abständen variieren, wobei letzteres insbesondere bei Transistoren zutreffend ist, ohne die Funktionalität hinsichtlich des Nachweises der Biomarker zu verlieren.

Die Elektroden sind im Rahmen der vorliegenden Erfindung in ihrer genauen Ausführung nicht beschränkt, sondern können übliche, fachbekannte Ausgestaltungen aufweisen. Die Elektrode an sich kann eine einfache Metallfläche sein, oder kann zum Beispiel auch eine auf oder aus einem Substrat geätzte Leiterbahn sein, oder ein Draht. Der Bereich der Elektrode, an den die Aptamere gebunden werden, kann dabei verschiedene Strukturen aufweisen, es ist lediglich notwendig, dass die resultierende Oberfläche dieses Elektrodenbereichs ausreichend ist, die gewünschte beziehungsweise notwendige Menge Aptamer zu binden.

Im Rahmen der vorliegenden Erfindung ist das oben und weiter unten beschriebene Verfahren, in welchem Elektroden mit den verschiedenen Aptamerlösungen inkubiert werden, nicht der einzige Weg, um verschiedene Aptamere zu immobilisieren. Dies kann in anderen bevorzugten Ausgestaltungen durch viele andere Verfahren, wie insbesondere elektrochemisches Molekülstripping, Drucken, Spotten oder Lithographie erreicht werden.

Die erfindungsgemäßen Aptamere werden bevorzugt über Bindungsgruppen an die Oberflächen der Elektroden gebunden. Die Bindungsgruppen können die Aptamere mittels Monothiolbindung oder über Multithiolbindungen, durch R-S-S-R' Gruppen, R-S-R' Gruppen, R-NH₂ Gruppen, R-CS₂ Gruppen, R-CN Gruppen, wobei R und R' = beliebige lineare aliphatische oder aromatische Moleküle mit einer Kettenlänge kürzer 100 nm, bevorzugt mit einer Kettenlänge kürzer 10 nm, wobei R von R' verschieden sein kann, Methyl-Carbodithioat-Ester, oder über andere kovalente Bindungen an der festen Phase/auf der Elektrode verankern bzw. immobilisieren, können aber auch über Oligonukleotide oder hochaffine Gruppen (Biotin-Streptavidin Kopplung) an die feste Phase, insbesondere Elektrode, verankern. Die Kopplung der Aptamere kann ebenfalls über Amid-Bindungen, carbonylähnliche Bildung von Carbamiden und Amiden, EsterGruppen erfolgen. Die Bindung kann über Klickreaktionen erfolgen (z.B. Azid-Alkin-Cycloaddition, Huisgen-Cycloaddition, Diels-Alder-Reaktion, Epoxidierungsreaktionen). Multithiolbindungen ergeben sich zum Beispiel aus Disulfidenhaltenden Phosphoramiditen (DTPA) wie 1,2-Diathian-4-O-Dimethoxytrityl 5-[(2-Cyano-ethyl)-N, N-Diisopropyl)]-Phosphoramidit, 1,2-Diathian-4-O-Dimethoxytrityl-5-Succinoyl-Iong-chain-Aminoalkyl, bis-Dithiolphosphoramidit oder tris-Dithiolphosphoramidit, oder mehrfache lineare Verknüpfungen dieser mit n = 2 - 10 (n=Anzahl der Einheiten) idealerweise n=3. Andere Moleküle mit mehrfachen Thioleinheiten sind ebenfalls als Bindungsgruppen geeignet.

Besonders bevorzugt sind HS-(CH₂)₆-Gruppen, die insbesondere am 5'-Ende des Aptamers gekoppelt sind (wodurch das Aptamer ein modifiziertes ist).

Weitere bevorzugte Gruppen sind Gruppen resultierend aus 1,2-Diathian-4-O-Dimethoxytrityl 5-[(2-Cyano-ethyl)-N, N-Diisopropyl)]-Phosphoramidit oder 1,2-Diathian-4-O-Dimethoxytrityl-5-Succinoyl-Iong-chain-Aminoalkyl.

Als Blockierungsmolekül, um Biofouling durch andere vorhandene Moleküle in den Proben zu vermeiden (unspezifische Bindung von Matrixkomponenten), wird erfindungsgemäß besonders bevorzugt Polyethylenglykolthiol-Lösung verwendet. Allerdings ist die vorliegende Erfindung nicht auf Polyethylenglykolthiol-Lösung beschränkt. Weitere einsetzbare Blockierungsmoleküle sind zum Beispiel 6-Mercapto-1-Hexanol, Rinderserum-Albumin oder Albumine anderer Tiere, Betaine, zwitterionische Phenyl-Schichten wie beispielsweise Phenyl-Phosphoryl-Cholin (PPC), Phenyl-Buttersäure, Poly(sulfobetain-3,4-ethyl-en-dioxythiophen), Polysulfobetain-Methacrylate, Polycarboxybetain-Methacrylate, oder deren Mischungen, Proteoglykane wie Lubricin, Hydrogele, 2-Methacryloyloxyethyl-Phosphorylcholine. Andere fachbekannte Blockierungsmoleküle sind einsetzbar.

In bevorzugten Ausführungsformen werden Polyethylenglykolthiol, 6-Mercapto-1-Hexanol oder längerkettige Moleküle gleicher Struktur sowie Rinderserum-Albumin verwendet.

In manchen Ausgestaltungen der vorliegenden Erfindung können zur Erhöhung der Dichte an Aptameren auf der Oberfläche der Elektroden leitfähige Polymere wie Poly(3,4-Ethylendioxythiophen)-Polystyrolsulfonat als eine Aptamer-einbettende Elektrodenbeschichtung eingesetzt werden. Es ist ebenfalls möglich, hierfür leitfähige 3-D Scaffolds, d.h. Mikro- und Nanogerüste, die aus Metallen, Kohlenstoff und leitfähigen Polymeren hergestellt werden, einzusetzen, wie zum Beispiel Nanomaterialien z.B. nanoporöses Gold, Platinblack, nanostrukturiertes Gold, MOFs, dendritische Moleküle, polyvalente Moleküle (vgl. auch weiter oben).

Im Fall der Verwendung der erfindungsgemäßen Biosensoren in Zusammenhang mit bzw. auf Multielektrodenfeldern können weitere Rezeptoren (beispielsweise Antikörper oder Aptamere) an der festen Phase bzw. auf den einzelnen Elektroden gebunden werden, die weitere relevante Biomarker binden. Bei diesen Biomarkern kann es sich um Biomarker für Demenzerkrankungen, speziell für Alzheimers Erkrankung handeln oder auch Neurotransmitter, andere Neurochemikalien, Hormone, oder Energieträgermoleküle.

Falls die verwendeten Sensoren OECT-Bauelemente sind, kann in einigen bevorzugten Ausführungsformen jedem Transistor ein Rezeptor, der den korrespondierenden Biomarker bindet und damit nachweist, zugeordnet werden. Gleiches gilt, wenn der Nachweis elektrochemisch erfolgt, und die Sensoren aus Elektrodenfeldern (Electrode Arrays) bestehen. In diesem Fall kann in einigen bevorzugten Ausführungsformen jeder Elektrode ein Rezeptor, der den korrespondierenden Biomarker bindet und damit nachweist, zugeordnet werden.

Wenn die Detektion über optische Bestimmung bzw. Verfahren erfolgt und die (modifizierten) Aptamere, insbesondere mit Fluorophoren modifizierten Aptamere, in Lösung vorliegen, können ebenfalls weitere Rezeptoren (beispielsweise Antikörper oder Aptamere), die weitere relevante Biomarker binden, mit in die Analytlösung gegeben werden, wenn diese mit weiteren Fluorophoren modifiziert wurden und bei anderen Wellenlängen angeregt werden bzw. Fluoreszenzstrahlung emittieren.

Bei den Biomarkern kann es sich um Tau Proteine, Amyloid-Peptide, saures Gliafaserprotein (GFAP), Apolipoprotein E, synaptosomal-assoziiertes Protein mit 25 kDa (SNAP-25), Wachstumsassoziiertes Protein 43 (Growth Associated Protein 43 - GAP-43), Beta-Sekretase (BACE1), Alzheimer-assoziiertes Neuronalstrang Protein (Alzheimer-Associated Neuronal Thread Protein - AD7C-NTP), Alpha-1 Antitrypsin (AAT), Amyloid-Precursor-Protein (APP), Neurogranin, Growth Differentiation Factor 15 (GDF15), Neuronenspezifische Enolase (NSE), Insulin Wachstumsfaktor Bindungsprotein 7 (Insulin Growth Factor Binding Protein 7 - IGFBP 7), TDP-43-Protein (transactive response DNA Binding Protein mit 43 kDa), Neuronal Pentraxin 2 (NPTX2), Soluble Triggering Receptor exprimiert an Myeloid Zellen 2 (sTREM2), Interleukin-6, Chitinase-3-artiges Protein 1 (CHI3L1/YKL-40), das APOE4 Gen oder andere handeln.

Es ist in einer bevorzugten Ausgestaltung bei der Anwendung der vorliegenden Erfindung in Assays bzw., anders ausgedrückt, bei Verwendung in Form von Elektrodenfeldern ebenfalls möglich, statt der gewünschten Aptamere oder anderer analytisch wirkender Aptamere oder Biomarker auf einzelne oder mehrere einzelne Elektroden andere Moleküle als Passivierungsmoleküle oder Rezeptoren ohne Bindungsaffinität aufzubringen. Elektroden, die mit diesen Molekülen modifiziert sind, können dann als Kontrollsensoren verwendet werden.

Weitere optische Assays können Enzyme-Linked Oligonucleotide Assays (ELONA, deutsch: enzymgekoppelter Oligonukleotidtest), Oberflächenplasmonresonanzassays, Lateral-Flow-Assays (deutsch: lateraler Flusstest), ALPHAScreen^{®} Assays, ALPHALisa^{®} Assays, Microscale Thermophoresis Assays (deutsch: mikroskaliger Thermophoresetest), oder elektrophoretische Mobilitäts-Verschiebungsassays sein.

In der vorliegenden Erfindung wurden insbesondere Möglichkeiten gefunden, NFL als AD-Biomarker in Blutproben mittels eines elektrochemischen Biosensors nachzuweisen. Diese basieren darauf, dass die NFL-Konzentration von AD-Patienten im Stuhl, Speichel, Urin, Blut, Serum bzw. CSF, insbesondere Blut, ansteigt schon bevor kognitive Einschränkungen evident werden. Diese erfindungsgemäßen Verfahren zum Nachweis (Tests) sind preiswert und minimalinvasiv, da nur eine kleine Menge (<10 ml) Speichel, Urin, Blut, Serum bzw. CSF, insbesondere Blut, benötigt wird. Die erfindungsgemäßen Verfahren zum Nachweis (Tests) können präventiv und dezentral bei einer breiten Bevölkerungsgruppe beim Arzt (Point-of-Care) durchgeführt werden, bevor kognitive Einschränkungen festgestellt werden.

Die erfindungsgemäßen Biosensoren verwenden einzelsträngige Desoxyribonukleinsäure (ssDNA) Moleküle als Aptamer-Rezeptoren, die selektiv NFL binden. Diese Aptamere werden bevorzugt durch in vitro Selektion (systematic evolution of ligands by exponential enrichment - SELEX) generiert. DNA-Aptamere besitzen gegenüber Antikörpern entscheidende Vorteile für deren Einsatz als Rezeptoren bei Biosensoren. So sind diese kleiner, thermisch stabiler, einfach chemisch modifizierbar und können preiswert synthetisch hergestellt werden. Die Aptamere werden im Rahmen der vorliegenden Erfindung auf Elektroden oder Transistoren oder anderen Signalwandlern immobilisiert, zusammen mit Molekülen, die die unspezifische Bindung von Komponenten der Probe (Blut, Serum, CSF, Urin, Speichel, Stuhl) unterdrücken. Der so erzeugte Biosensor wird der Probe ausgesetzt, wodurch das darin enthaltene NFL an die Aptamere, die an den Signalwandler gebunden sind, bindet. Der Signalwandler registriert diesen Bindungsvorgang und wandelt diese in ein quantifizierbares elektrisches Signal um. Dieses kann automatisiert oder von einem Nutzer, bevorzugt einem Arzt, für die Diagnose verwendet werden. Bei dem Signalwandler kann es sich in Ausführungsformen der vorliegenden Erfindung auch um ein Mehrkanal(elektroden)system (Array) handeln, auf dem verschiedene Aptamerrezeptoren für NFL oder weitere AD-Biomarker, wie z.B. Amyloid-beta-Oligomere, Tau-Protein oder andere immobilisiert und nachgewiesen werden können.

Im Rahmen der vorliegenden Erfindung wird also davon ausgegangen, dass mindestens einen Aptamerrezeptor für NFL, im Rahmen der vorliegenden Erfindung auch einfach als Aptamer bezeichnet, der durch SELEX bestimmt wird und der selektiv an NFL bindet, verwendet wird. Die Aptamerrezeptoren (Aptamere) werden im Rahmen der vorliegenden Erfindung mit mindestens einem Signalwandler kombiniert, der die Bindung des Aptamers an NFL-Moleküle detektieren und in ein elektrisches Signal umwandeln kann. Der Signalwandler darf dabei im Rahmen der vorliegenden Erfindung nicht durch Matrixbestandteile der Probe beeinflusst werden.

Die erfindungsgemäßen Aptamere können weiterhin für die in oder für die Herstellung von erfindungsgemäßen Biosensoren genutzt werden, die nicht auf elektrochemischen, sondern auf optischen, magnetischen oder gravimetrischen oder anderen Transducerprinzipien (Transducer = Signalwandler) beruhen. Weiterhin können die erfindungsgemäßen Aptamere im Wesentlichen für alle Formen der Anwendung genutzt werden, die schon für Antikörper etabliert wurden, z.B. als Therapeutika oder als Affinitätstag.

Die vorliegende Erfindung betrifft demgemäß in einem Aspekt Aptamermoleküle, insbesondere beruhend auf einzelsträngiger Desoxyribonukleinsäure, für die Bindung an Neurofilament Light (NFL), sowie die Nutzung dieser Aptamermoleküle als Rezeptor in Biosensoren. Diese Biosensoren können für die Erkennung der Degradation von neuronalem Gewebe genutzt werden, insbesondere bei der Früherkennung von neurodegenerativen Prozessen, wie sie bei der Alzheimererkrankung und anderen Demenzerkrankungen auftreten, indem mit Ihnen Neurofilament Light als Biomarker detektiert wird. Der Nachweis der Biomarker kann in Körperflüssigkeiten und/oder Stuhl, bevorzugt Speichel, Urin, Blut, Serum oder CSF, insbesondere Blut, erfolgen. In dieser Anmeldung wird NFL als AD-Biomarker in Körperflüssigkeiten und/oder Stuhl, bevorzugt Speichel, Urin, Blut, Serum oder CSF, insbesondere Blutproben, mittels eines Biosensors nachgewiesen, da die NFL-Konzentration von AD-Patienten in Körperflüssigkeiten und/oder Stuhl, bevorzugt Speichel, Urin, Blut, Serum oder CSF, insbesondere Blut, ansteigt, schon bevor kognitive Einschränkungen evident werden. Der erfindungsgemäße Biosensor verwendet einzelsträngige Desoxyribonukleinsäure (ssDNA) Moleküle, die selektiv NFL binden, als Aptamer-Rezeptoren. Diese Aptamere werden im Rahmen der vorliegenden Erfindung durch in vitro Selektion (systematic evolution of ligands by exponential enrichment - SELEX) generiert. DNA-Aptamere besitzen gegenüber Antikörpern entscheidende Vorteile für deren Einsatz als Rezeptoren bei der Entwicklung von Biosensoren. So sind diese kleiner, thermisch stabiler, einfach chemisch modifizierbar und können preiswert synthetisch hergestellt werden. Da Aptamere durch chemische Synthese und nicht durch biologische Prozesse wir Zellkulturexpression gewonnen werden, können bakterielle und virale Kontaminationen vermieden, große Synthesemengen realisiert und Chargenvariationen reduziert werden. Aptamere erzeugen geringere Immunreaktionen und die geringe Größe (<30 kDa gegenüber ~150 kDa für Antikörper) machen sie zugänglich zu Epitopen am Zielmolekül, an die Antikörper aus sterischen Gründen nicht binden können. Konformale Änderungen in der Struktur der Aptamere infolge langer Lagerungen können durch einfache Aufwärm- und Abkühlprotokolle zurückgesetzt werden, wodurch sich deren Lagerfähigkeit verbessert und Lager- und Transportkosten reduziert werden können.

Trotz der robusten und einfachen Struktur der Aptamere können diese mit hoher Spezifität, Selektivität und Affinität an ihre Zielmoleküle binden.

Die Aptamere oder modifizierten Aptamere können erfindungsgemäß (im Bindungsassay) frei in der Probenlösung vorliegen oder sie können auf Elektroden, Transistoren, Nanomaterialien oder anderen Signalwandlern immobilisiert vorliegen. Jeweils zusammen mit Molekülen, die die unspezifische Bindung von Komponenten der Probe (Stuhl, Speichel, Urin, Blut, Serum, CSF) unterdrücken. Zudem können die Aptamere mit Sondenmolekülen modifiziert werden, wie Fluorophoren, Nanomaterialien, Enzymen oder Redoxmolekülen, um eine Signalwandlung zu ermöglichen oder zu verstärken. Zudem können Bindungsgruppen oder andere funktionelle Gruppen an die Aptamere konjugiert werden. Der so erzeugte erfindungsgemäße Biosensor wird erfindungsgemäß dann der Probe ausgesetzt, wodurch das darin enthaltene NFL an die Aptamere bindet, die an einen Signalwandler immobilisiert sein können.

Ein Nachweis des NFL-Biomarkers in Körperflüssigkeiten und/oder Stuhl mittels des erfindungsgemäßen Verfahrens (Tests) ist preiswert und minimalinvasiv, da nur eine kleine Menge (<10 ml) in Körperflüssigkeiten und/oder Stuhl, bevorzugt Speichel, Urin, Blut, Serum oder CSF, insbesondere Blut, (für den Assay) benötigt wird. Das erfindungsgemäße Verfahren (Test) kann potentiell präventiv und dezentral bei einer breiten Bevölkerungsgruppe beim Arzt (Point-of-Care) durchgeführt werden, bevor kognitive Einschränkungen festgestellt werden.

Die erfindungsgemäßen Aptamere können, unter anderem und bevorzugt, für die erfindungsgemäßen elektrochemischen, optischen, elektrischen, magnetischen, gravimetrischen Biosensoren oder Sensoren, die auf anderer Transducerprinzipien (Transducer = Signalwandler) beruhen, eingesetzt werden.

Bevorzugt im Rahmen der vorliegenden Erfindung sind optische, elektrochemische und elektrische (OECT) Transducer.

Weiterhin können die erfindungsgemäßen Aptamere potentiell für alle Formen der Anwendung genutzt werden, die schon für Antikörper etabliert wurden, z.B. als Therapeutika, als Affinitätstag, zum Markieren von Systemen die NFL enthalten, oder zur Qualitätskontrolle. Als Oligonukleotide können die ssDNA-Aptamere als Reagenzien für Technologien verwendet werden, die auf Nukleinsäure-basierten Systemen beruhen wie DNA-Nanotechnologie, DNA-Pharmazeutika, DNA-Computing oder andere.

Elektrochemische Biosensoren sind aufgrund ihrer hohen Empfindlichkeit, der einfachen Handhabung und Herstellung, der Möglichkeit der Miniaturisierung der Elektroden und der Parallelisierbarkeit zur gleichzeitigen (bzw. multiplexed) Detektion redundanter bzw. komplementärer Sensorsignale anderen Sensorsystemen überlegen und eignen sich daher insbesondere für Point-of-Care Anwendungen. Ähnliches gilt für Sensoren basierend auf OECTs, nur das diese als Bauelement drei Elektroden enthalten und niedrigere Nachweisgrenzen ermöglichen.

Demgemäß sind erfindungsgemäß optische Sensoren, elektrochemische Biosensoren und Sensoren basierend auf OECT besonders bevorzugt. Die vorliegende Erfindung ist jedoch nicht auf diese beschränkt.

Die erfindungsgemäßen Sensoren bzw. Biosensoren, bis auf den optischen Sensor, beruhen auf Metallelektroden, die als Einzelbauelement oder als Bauelementfelder vorliegen können. Auf diesen Elektroden werden erfindungsgemäß die Aptamermoleküle, die NFL binden, immobilisiert. Im Fall der Verwendung von Bauelementfeldern können weitere Rezeptoren (beispielsweise Antikörper oder Aptamere) auf den einzelnen Bauelementen gebunden werden, die weitere relevante Biomarker binden, siehe zum Beispiel, aber nicht ausschließlich, die Beispiele. Neben den Aptameren werden in bevorzugten Ausführungsformen Blocking-Moleküle an die Elektroden gebunden, die eine unspezifische Bindung von Matrixmolekülen unterdrücken. Bei Kontakt des Sensors mit der zu untersuchenden Probe (Körperflüssigkeit und/oder Stuhl, beispielsweise Speichel, Urin, CSF, Blut oder Serum, insbesondere Blut) wird die Bindung des NFL an die Aptamermoleküle elektrochemisch/elektrisch gemessen.

Die vorliegende Erfindung betrifft in einem Gegenstand bestimmte ssDNA-Moleküle, die als Aptamer verwendet werden mit einer definierten Sequenz an Desoxyribonukleotiden, die in der Lage sind Neurofilament Light mit hoher Spezifität und Affinität zu binden. Diese Moleküle wurden aus einer ssDNA-Bibliothek selektiert indem die Bibliothek mit NFL inkubiert wurde. Die bindenden ssDNA-Moleküle wurden durch Kapillarelektrophorese von nichtbindenden ssDNA-Molekülen getrennt. Die bindenden ssDNA-Moleküle wurden anschließend isoliert und amplifiziert. Die Sequenz der Aptamere wurde durch Sequenzierung und anschließender in-silico Analyse aus den bindenden ssDNA-Moleküle abgeleitet.

Die Aptamere sind zusammengesetzt aus Nukleotiden Adenin (A), Guanin (G), Cytosin (C), Uracil (U) und Thymin (T) bzw. Derivaten dieser Nukleotide mit chemischen Modifizierungen wie z.B. Methylierungen, Spiegelmere der Nukleotide oder andere Zucker-modifizierte Nukleotide, Nukleobasen-modifizierte Nukleotide und Rückgratmodifizierte Nukleotide.

Der Begriff "Derivat" bezeichnet im Zusammenhang mit der vorliegenden Erfindung in einigen bevorzugten Ausführungsformen ein Aptamer, das an der Nukleobase, an der Pentose oder am Phosphat-Rückgrat chemisch modifiziert ist und das eine chemische Struktur aufweist, die in natürlicher DNA oder RNA nicht vorkommt. Insbesondere bezeichnet der Begriff Derivat ein Aptamer, das eine chemische Struktur enthält, die von Desoxyribose, Ribose, Phosphat, Adenin, Guanin, Cytosin, Thymin, oder Uracil abweichend ist. Ein Aptamer-Derivat kann an der Nukleobase, an der Pentose oder am Phosphat-Rückgrat modifiziert sein. Der Begriff "Derivat" kann im Rahmen der vorliegenden Erfindung ein Aptamer bezeichnen, bei dem natürlich in DNA und RNA vorkommende Nukleotide teilweise oder vollständig durch chemisch davon abweichende Nukleotide, sogenannte modifizierte Nukleotide, ersetzt sind und/oder dessen molekulare Struktur anderweitig modifiziert ist, insbesondere durch Anbindung nicht natürlich in RNA oder DNA vorkommender Strukturen, und/oder die ein modifiziertes Rückgrat aufweisen.

Einige, nicht einschränkende, spezielle Beispiele für Derivate im Rahmen der vorliegenden Erfindung sind:
- Aptamere, die an mindestens einem Nukleotid eine Alkylierung, Arylierung oder Acetylierung, Alkoxylierung, Halogenierung, eine Aminogruppe oder eine andere funktionelle Gruppe aufweisen;
- Aptamere, die eine Basenmodifikation aufweisen, wie Bromuridin, insbesondere Modifikationen, die die Hydrophobizität erhöhen;
- markierte Aptamere, auch bezeichnet als gelabelte Aptamere, wobei bevorzugte Markierungen (Label) visuell, optisch, photonisch, elektronisch, akustisch, opto-akustisch, nach Masse, elektrochemisch, elektrooptisch, spektrometrisch, enzymatisch, oder anderweitig chemisch, biochemisch oder physikalisch detektierbar sind, und wobei Label beispielsweise angebundene Reporter-, Marker- oder Adaptermoleküle sein können;
- Aptamere, die enantiomere Nukleotide aufweisen;
- Aptamere, die ganz oder teilweise als Phosphorthioat-RNA oder -DNA, Phosphordithioat-RNA oder -DNA, Phosphorselenoat-RNA oder -DNA, Phosphordiselenoat-RNA oder -DNA, Phosphoroamidat-RNA oder -DNA, locked nucleic acid (LNA), peptide nucleic acid (PNA), N3'-P5' Phosphoramidat RNA/DNA, Cyclohexennukleinsäure (CeNA), Tricyclo-DNA (tcDNA) oder Spiegelmer vorliegen, oder die Phosphoramidatmorpholin (PMO) Komponenten aufweisen.
- Aptamere, die ganz oder teilweise Modifizierungen der Ribose aufweisen wie 2'Fluoro Nukleinsäuren, 2'-Fluoro Arabino Nukleinsäuren, 2',2'-Difluorocytidine, geschlossene Nukleinsäuren, unverschlossene Nukleinsäuren, Xenogenische Nukleinsäuren.
- Aptamere, die ganz oder teilweise aus künstlichen Nukleotiden bestehen wie (7- (2-Thienyl) Imidazo [4, 5-b] Pyridin), 6-Amino-5-Nitro-3- (1'-β-D-2'-Deoxyribofuranosyl)-2 (1H)-Pyridon, 2-Amino-8- (1'-β-D-2'-Deoxyribofuranosyl)-Imidazo-[1,2-a]-1,3,5-Triazin-4 .

Beispiele für markierte Aptamere, sind solche, deren Markierung durch Lumineszenz, UV/VIS-Farbgebung, enzymatisch, elektrochemisch, immunologisch oder radioaktiv nachgewiesen werden kann. Erfindungsgemäß einsetzbare Markierungsmittel sind bevorzugt Lumineszenz-, Phosphoreszenz, Fluoreszenz-, radioaktive, oder UV/VIS-Markierungssubstanzen, die, wie dem Fachmann bekannt, insbesondere als Reporter, Marker- oder Adaptermoleküle an die Aptamere binden.

Beispiele für einsetzbare Markierungsmittel/Markierungsstoffe, wobei diese Aufzählung nicht exklusiv ist, sind:
- Photolumineszente Stoffe, insbesondere Fluorophore, wie, zum Beispiel, Bisbenzimidazol, Fluoreszein, Acridinorange, Cy5, Cy3, Propidiumiodid, Tetramethyl-6-Carboxyrhodamin (TAMRA), Texas Red^{®}, Rhodamin, Quantum Dots; chemilumineszente Stoffe, die sichtbares Licht als Folge einer chemischen Reaktion aussenden; biolumineszente Stoffe, die sichtbares Lichts als Folge einer Enzymreaktion, beispielsweise durch Luciferase katalysierte Redoxreaktion, aussenden; wobei die Auswertung zum Beispiel visuell oder mit entsprechenden Messgeräten, beispielsweise Multilabel-Counter, Fluoreszenzmikroskop, oder durch Durchflusszytometrie, beispielsweise Cytofluorimeter, erfolgen kann.
- Katalysatoren, kolloidale metallische Teilchen, beispielsweise Gold-Nanopartikel, kolloidale nicht metallische Teilchen, Quantum Dots, organische Polymere, Latexteilchen, Nanofasern, insbesondere Kohlenstoffnanofasern, Nanoröhren, insbesondere Kohlenstoffnanoröhren, Dendrimere, Proteine oder Liposome mit signalerzeugenden Stoffen, wobei dann der Nachweis kolorimetrisch erfolgen kann, insbesondere im Falle von kolloidalen Teilchen.
- Visuell detektierbare Farbstoffe, wie beispielsweise interkalierende Farbstoffe.
- Enzyme, deren enzymatische Reaktion durch den Verbrauch oder die Entstehung detektierbarer Substrate oder Produkte gekennzeichnet ist, was dann beispielsweise optisch oder elektrochemisch detektiert werden kann. Ein Nachweis kann beispielsweise mit Enzymen als Markierungssubstanzen geführt werden, die Substrate zu farbigen Produkten umsetzen, vorzugsweise Peroxidase, Green Fluorescent Protein (GFP), Luciferase, [beta]-Galactosidase oder alkalische Phosphatase.

Die folgende Tabelle 1 gibt die optimierten erfindungsgemäßen Aptamere an:

**Tabelle 1**

| Aptamere | | | | | | | | | |
|---|---|---|---|---|---|---|---|---|---|
| SEQ ID NO: | **Sequenz** | Rang.1R | Anzahl.1R | RPM.1R | Rang.3R | Anzahl.3R | RPM.3R | Anreicherung_ 3R&1R | log2E_ 3R&1R |
| 1 | GCATACTTGCGAGTCCTAACAGGTATTGGTCAAGGTACGATTG | 228038 | 2 | 1.8 | 1 | 1189 | 1773.83 | 985.461 | 9.945 |
| 2 | TCCCTCCATCTAAGCCTTTATTTCGTATGTCGTCGAAGGTGTG | 502225 | 1 | 0.9 | 9 | 299 | 446.07 | 495.633 | 8.953 |
| 3 | CATAGCCCGCGCAATCTACGTAGACACACCTAACGAAACCAAC | 192297 | 2 | 1.8 | 8 | 329 | 490.82 | 272.678 | 8.091 |

### Aptamer Sequenzen:

Aptamer FZJ-NFL_1 (Aptamer 1), GCA TAC TTG CGA GTC CTA ACA GGT ATT GGT CAA GGT ACG ATT G
Aptamer FZJ-NFL_2 (Aptamer 2), TCC CTC CAT CTA AGC CTT TAT TTC GTA TGT CGT CGA AGG TGT G
Aptamer FZJ-NFL_3 (Aptamer 3), CAT AGC CCG CGC AAT CTA CGT AGA CAC ACC TAA CGA AAC CAA C

Diese Aptamere können 1, 2, 3, 4, 5, 6, 7, 8, 9, 10, 11, 12, 13, 14, 15, 16, 17, 18 oder 19 Modifizierungen in der Sequenz aufweisen, um deren Spezifität oder Affinität zu erhöhen. Diese Modifizierungen können das Entfernen, Substituieren und/oder Erweitern der ursprünglichen Sequenz einschließen, bzw. substituiert, deletiert und/oder insertiert sind. Dies kann zu Sequenzen führen, die 95%, 90%, 85%, 80%, 75%, 70%, 65%, 60%, oder 50% Übereinstimmung in der Sequenz mit den ursprünglichen Aptameren aufweisen.

Besonders geschützt sind die Stem-Loop Strukturen (bei einsträngiger DNA bzw. RNA vorkommende sekundäre Stamm-Schleife-Strukturen (englisch stem-loop) mit doppelsträngig ausgebildetem Stamm und kurzer einzelsträngiger Schleife; auch Haarnadelstrukturen genannt), da diese entscheidend an der Bindung zu NFL beteiligt sind.

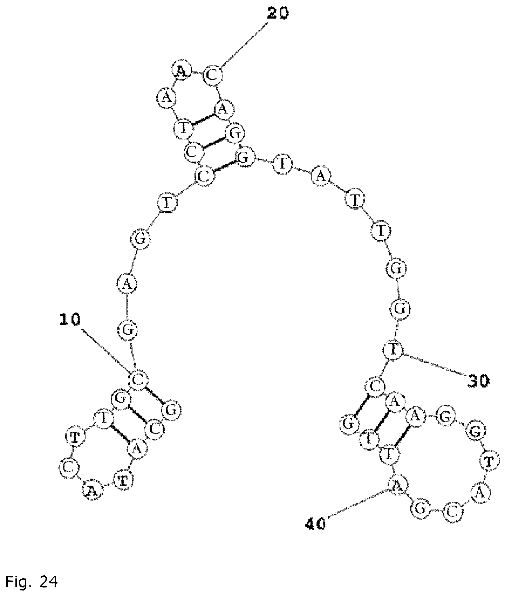

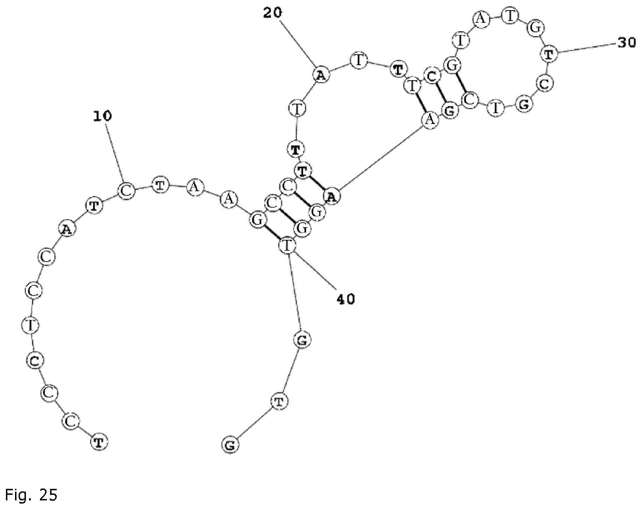

Aptamer FZJ-NFL_1, weist drei Stem-Loop Strukturen auf. Die Nummerierung startet vom 5' Ende und setzt sich zum 3' Ende fort. Entsprechend sind besonders Nukleotide 1 bis 10 (GCATACTTGC), Nukleotide 15 bis 23 (CCTAACAGG), Nukleotide 31 bis 43 (CAAGGTACGATTG) für die Funktionalität des Aptamers entscheidend, siehe Fig. 24. Die Stem-Loop Strukturen von FZJ-NFL_2 umfassen die Nukleotide 14 bis 40, aber Nukleotide 18 bis 22 weisen keine Basenpaarung auf und sind besonders geschützt, siehe Fig. 25.

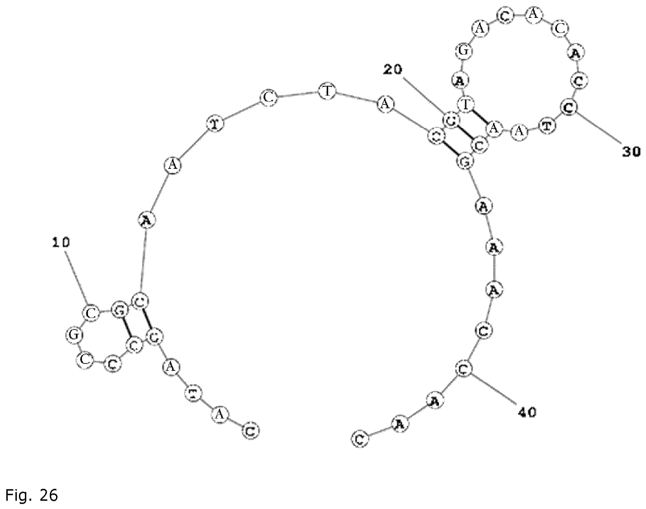

Die Stem-Loop Strukturen von Aptamer FZJ-NFL_3 kann zwei Stem-Loop Strukturen ausbilden, welche Nukleotide 5 bis 12 (GCCCGCGC) sowie Nukleotide 19 bis 35 (CGTAGACACACCTAACG) umfassen, welche besonders geschützt sind, siehe Fig. 26.

In weiteren Ausführungsformen der vorliegenden Erfindung ist das Aptamer der SEQ ID No:1 besonders bevorzugt. Denn es wurde gefunden, dass in diesem Aptamer ein Sequenzmotiv umfassend die Nukleotide 23 bis 32 (GT ATT GGT CA) vorkommt, welches an der Bindung mit dem NFL beteiligt ist.

Dieses Sequenzmotiv, d.h. die Nukleotide 23 bis 32 (GT ATT GGT CA), und die Sequenz GTATTGGTCA sind weitere Gegenstände der vorliegenden Erfindung.

Entsprechend ist auch Gegenstand der vorliegenden Erfindung die Verwendung eines Sequenzmotivs umfassend die Nukleotide 23 bis 32 aus SEQ ID NO: 1, in der dortigen Abfolge, d.h. GTATTGGTCA, zur Bindung eines dieses Sequenzmotiv enthaltenden Aptamers an Neurofilament Light.

Zudem ist auch Gegenstand der vorliegenden Erfindung die Verwendung eines Sequenzmotivs mit den Nukleotiden GTATTGGTCA, in dieser Abfolge, zur Bindung eines dieses Sequenzmotiv enthaltenden Aptamers an Neurofilament light.

Genauso ist ein Peptid umfassend die Nukleotidsequenz GTATTGGTCA oder bestehend aus der Nukleotidsequenz GTATTGGTCA Gegenstand der vorliegenden Erfindung. Die Nukleotidsequenz GTATTGGTCA, deren Bedeutung für die Bindung an Neurofilament Light neu gefunden wurde, ist also für sich allein oder in Form eines Oligonukleotids umfassend diese oder bestehend aus dieser Gegenstand der vorliegenden Erfindung.

Diese erfindungsgemäßen Aptamere können weitere funktionale Gruppen enthalten, die vorzugsweisen am 3' oder 5'-Ende des Aptamers angebracht sind bzw. sich an oder zwischen (interkalant) den Nukleotiden befinden können. Bei diesen Molekülen kann es sich um Sondenmoleküle wie Fluorophore, Nanomaterialien (Silbernanopartikel, Platinnanopartikel, Quantumdots, magnetische Nanopartikel u.a.), Enzyme (Meerrettich-Peroxidase, Glukoseoxidase und andere) oder Redoxmoleküle (Ferrocen, Methylenblau, Anthrachinon u.a.) handeln, die die Anwesenheit des Aptamers anzeigen, die eine Signalwandlung ermöglichen, das Signal verstärken oder schwächen.

Beispiele für erfindungsgemäß bevorzugt einsetzbare Fluorophore sind solche ausgewählt aus der Gruppe bestehend aus Cyanin 5 (Cy5), grün fluoreszierendes Protein (GFP), der Gruppe der Xanthen-Farbstoffe, Texas Red, Quantumdots, Fluorescein und dessen Derivate, G-Dye100, Nilblau, sowie Mischungen davon, besonders bevorzugt Cyanin 5.

Beispiele für erfindungsgemäß bevorzugt einsetzbare Nanomaterialien sind solche ausgewählt aus der Gruppe bestehend aus Silbernanopartikeln, Nanopartikeln mit katalytischen Eigenschaften bestehend aus Platin, Iridium, Cobalt, Ruthenium, Nickel, Cer, Quantumdots, magnetischen Nanopartikeln, Photonen-hochkonvertierende Nanopartikel wie NaYF₄:Yb/Er und NaYF₄:Yb/Tm und Mischungen davon, bevorzugt magnetischen Nanopartikeln aus Fe₃O₄ oder andere Stöchiometrie, MeFe₂O₄ (Me=Co, Cu, Mn, Zn, Ni), Metallnanopartikel aus Fe, Ni, Co, Pd, oder einer dieser Partikel dekoriert mit jeweils einem Metall von Fe, Ni, Co, Pd, oder einer dieser Partikel dekoriert mit organischen Molekülen, Biomolekülen, Polymeren oder anorganischen Filmen wie SiOx, AlOx, TiOx.

Beispiele für erfindungsgemäß bevorzugt einsetzbare Enzyme sind solche ausgewählt aus der Gruppe bestehend aus Meerrettich-Peroxidase, Glukoseoxidase, Phosphatase, Ferredoxin, Hydrogenase und Mischungen davon, bevorzugt Meerrettich-Peroxidase. Beispiele für erfindungsgemäß bevorzugt einsetzbare Redoxmoleküle sind solche ausgewählt aus der Gruppe bestehend aus Ferrocen, Methylenblau, Anthrachinon, Nilblau, Phenazin-Ethosulfat, organometalische Komplexe wie Porphyrine, Cyanine, [Os(4,4'-Dimethyl-2,2-dipyridil) 2 Cl(4-(aminomethyl)Pyridin)], Tetrathiafulvalen und Mischungen davon, bevorzugt Methylenblau.

Beispiele für erfindungsgemäß bevorzugt einsetzbare Bindungsgruppen sind solche ausgewählt aus der Gruppe bestehend aus Monothiolen (z.B. Alkanthiolen HS-(CH₂)ₙ-), Multithiolen, Disulfiden R-S-S-R', R-S-R' Gruppen, R-NH₂ Gruppen, R-CS₂ Gruppen, R-CN Gruppen, wobei R und R'= beliebige lineare aliphatische oder aromatische Moleküle mit einer Kettenlänge kürzer 100 nm, besonders bevorzugt mit einer Kettenlänge kürzer 10 nm, wobei R von R' verschieden sein kann, Methyl-Carbodithioat-Estern, gegebenenfalls kovalenter Bindung, und Mischungen davon, bevorzugt Multithiolbindungen wie bis-Dithiolphosphoramidit oder tris-Dithiolphosphoramidit, oder auch 1,2-Diathian-4-O-Dimethoxytrityl 5-[(2-Cyanoethyl)-N, N-Diisopropyl)]-Phosphoramidit, 1,2-Diathian-4-O-Dimethoxytrityl-5-Succinoyl-Iong-chain-Aminoalkyl. Es sei darauf hingewiesen, dass dies zwar bevorzugte Bindungsgruppen sind, im Rahmen der vorliegenden Erfindung jedoch auch weitere Möglichkeiten zur Bindung an feste Phase möglich sind - vgl. weiter oben.

Ein Beispiel für eine besonders bevorzugte Bindungsgruppe ist HS-(CH₂)₆.

Andere Beispiele für bevorzugte Bindungsruppen sind Gruppen resultierend aus 1,2-Diathian-4-O-Dimethoxytrityl 5-[(2-Cyano-ethyl)-N, N-Diisopropyl)]-Phosphoramidit oder 1,2-Diathian-4-O-Dimethoxytrityl-5-Succinoyl-Iong-chain-Aminoalkyl.

Zudem können diese Bindungsgruppen mit einzelsträngigen DNA-Molekülen an die Sensoroberfläche gebunden werden, die komplementär oder teilkomplementär zur Aptamersequenz sind und mit diesem hybridisieren und somit eine Immobilisierung des Aptamers ermöglichen. Diese Hybridisierung kann so ausgestaltet werden, dass diese wieder gebrochen wird und eine Separation des Aptamers erfolgt, wenn das Zielmolekül gebunden wird. Die Hybridisierung von komplementären oder teilkomplementären einzelsträngigen DNA-Molekülen kann auch erfolgen, wenn der Aptamer direkt an die Sensoroberfläche hybridisiert wurde

Weiterhin betrifft die vorliegende Erfindung Sensoren zum Nachweis von NFL. Die erfindungsgemäßen Sensoren enthalten Aptamer FZJ-NFL_1, 2, oder 3 oder Kombinationen von diesen. Wird NFL gebunden, so wird ein Signal erzeugt, welches einfach qualitativ ausgewertet werden oder zusätzlich oder alternativ quantifiziert werden kann.

Die erfindungsgemäßen Biomarkersensoren können Verwendung finden in automatisierten Multititerplatten.

Die Proben, bevorzugt Körperflüssigkeiten und/oder Stuhl, bevorzugt Speichel, Urin, Blut, Serum oder CSF, insbesondere Blut, können vor Einsatz in dem Verfahren der vorliegenden Erfindung entsprechend den in der Fachwelt üblichen Verfahren aufgearbeitet bzw. vorbereitet werden.

Es sei darauf hingewiesen, dass das Verfahren der vorliegenden Erfindung insbesondere dafür geeignet und dazu gedacht ist, Körperflüssigkeitsproben, insbesondere Blut, zu untersuchen, die bereits als solche vorliegen, d.h. die Entnahme von Körperflüssigkeiten und/oder Stuhl ist nicht Teil des Verfahrens der vorliegenden Erfindung.

Die vorliegende Erfindung bezieht sich auf Proben, die bevorzugt Körperflüssigkeiten und/oder Stuhl, besonders bevorzugt Speichel, Urin, Serum, Blut und Cerebrospinalflüssigkeit (CSF), insbesondere Blut, sind. In alternativen Ausführungsformen bezieht sich die Erfindung auf Proben, die bevorzugt Körperflüssigkeiten, besonders bevorzugt Serum, Blut und Cerebrospinalflüssigkeit (CSF), insbesondere Blut, sind (also ohne Stuhl, Speichel, Urin), wobei diese alternativen Ausführungsformen gleichwertig sind und nur der Einfachheit halber nicht jeweils mit angeführt wurden; die Definition der Proben dieser alternativen Ausführungsformen können also jeweils die etwas bereitere Definition ersetzen.

Einige, jedoch nicht alle, Vorteile der vorliegenden Erfindung sind die Folgenden:
- Das erfindungsgemäße Verfahren ist minimalinvasiv. Die für den Test benötigte Körperflüssigkeit kann zum Beispiel auch bei einer aus anderem Grunde durchgeführten Flüssigkeitsentnahme (Blutabnahme) mit entnommen werden.
- Das erfindungsgemäße Verfahren ist preiswert.
- Das erfindungsgemäße Verfahren ist kann präventiv durchgeführt werden.
- Das erfindungsgemäße Verfahren ist kann dezentral sogar bei breiten Bevölkerungsgruppen beim Arzt (Point-of-Care) durchgeführt werden.
- Die erfindungsgemäßen Aptamere wiesen eine hohe Spezifität und Affinität für Neufilament Light auf.
- Die erfindungsgemäßen Aptamere sind hoch selektiv gegenüber Neurofilament Light.
- Die erfindungsgemäßen Aptamere weisen hohe chemische Stabilität auf.
- Die erfindungsgemäßen Aptamere zeigen geringe Immunogenität.
- Die erfindungsgemäßen Aptamere

Im Rahmen der vorliegenden Erfindung werden mithin neben dem erfindungsgemäßen Verfahren sowohl Aptamermoleküle bereitgestellt, die selektiv und mit hoher Affinität an Neurofilament Light binden, als auch Sensoren und Assays für den Nachweis von Neurofilament Light. Diese erfindungsgemäßen Aptamermoleküle, Sensoren und Assays, sowie das erfindungsgemäße Verfahren auch, sind am Point-of-Care verwendbar, kostengünstig herzustellen, weisen hohe Stabilität auf, sind einfach zu bedienen und weisen eine hohe Testgenauigkeit auf; sind mithin besonders vorteilhaft.

Im Folgenden wird die Erfindung unter Bezugnahme auf die Figuren näher erläutert. Die Figuren sind dabei nicht unbedingt maßstabsgetreu und vereinfacht. So sind übliche, dem Fachmann geläufige Maßnahmen und Vorrichtungselemente etc. nicht unbedingt dargestellt (Verschaltung, Wände, genaue Molekülstruktur etc.), um die Lesbarkeit der Figuren zu erleichtern.

### Figurenbeschreibung:

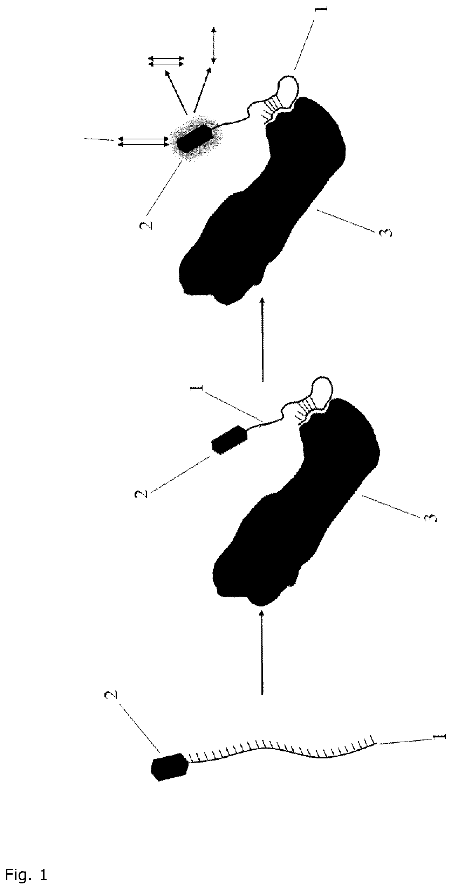

Fig.1 zeigt ein Schema für ein optisches Assay gemäß der vorliegenden Erfindung und die dazugehörigen Komponenten bestehend aus Aptamer 1 mit Fluorophor 2 und Analyt NFL 3. Bindet das Aptamer 1 an das NFL 3, so ändert sich die Polarisation der Fluoreszenz des Fluorophors 2, was detektierbar ist und als Messsignal dient.

In dieser Figur ist dargestellt, dass an das 3'-Ende des Aptamers ein Fluorophor bindet und über die Bindung an NFL dieses fluoreszenzmarkiert. Eine mögliche Verbindung zwischen Aptamer und Elektrodenoberfläche über das 5'-Ende wird in anderen Figuren illustriert.

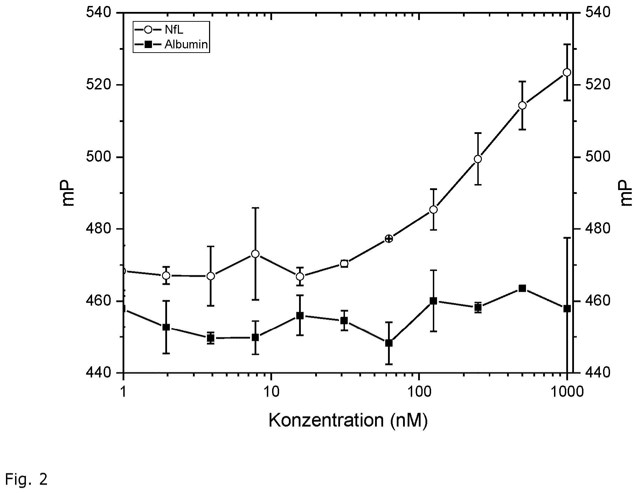

Fig. 2 zeigt eine Kalibrierkurve des optischen Assays für das erfindungsgemäße, modifizierte Aptamer_ FZJ-NFL_opt1 gemäß Beispiel 3, aufgenommen durch Fluoreszenzpolarisation als Funktion der Konzentration. Der lineare Detektionsbereich des optischen Assays startet von etwa 17 nM und reicht bis 1000 nM, der K_{D}-Wert beträgt 181,73 nM (Fig. 2, obere Kurve). Das modifizierte Aptamer_ FZJ-NFL_opt1 zeigt keine Bindungsaffinität zu Humanserumalbumin, was die hohe Spezifität der Aptamere unterstreicht (Fig. 2 untere Kurve).

Die Kalibrierung erfolgt dabei indem erst die Polarisation des Fluoreszenzlichtes für verschiedene Konzentrationen des Analyten (NFL) gemessen und mithilfe der Hill-Gleichung gefittet wird. Durch anschließende Messung der Polarisation des Fluoreszenzlichtes einer Lösung unbekannter Konzentration, kann mithilfe der gefitteten Funktion die Konzentration bestimmt werden.

*K*_{D} ist die Dissoziationskonstante, die durch fitten der Kurve mittels eines geeigneten Modells, bevorzugt der Hill Gleichung, erhalten wurde. Der aufgetragene mP-Wert ist ein dimensionsloses Maß für die Polarisation des Fluoreszenzlichtes und wird auch als Millipolarisation bezeichnet. Die Ergebnisse belegen die hohe Affinität und Spezifität des optischen Assays.

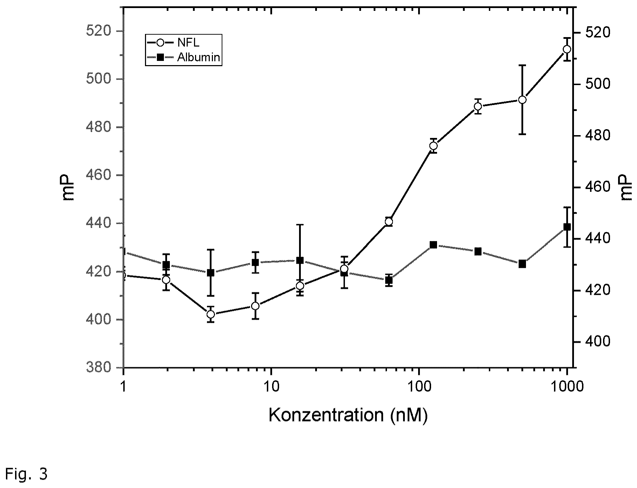

Fig. 3 zeigt eine Kalibrierkurve des optischen Assays für das erfindungsgemäße, modifizierte Aptamer_ FZJ-NFL_opt2 gemäß Beispiel 3, aufgenommen durch Fluoreszenzpolarisation als Funktion der Konzentration. Der lineare Detektionsbereich des optischen Assays startet von etwa 30 nM und reicht bis 1000 nM, der K_{D}-Wert beträgt 100,25 nM für Aptamer_ FZJ-NFL_opt2 (Fig. 3 obere Kurve). Das modifizierte Aptamer_ FZJ-NFL_opt2 zeigt keine Bindungsaffinität zu Humanserumalbumin, was die hohe Spezifität der Aptamere unterstreicht (Fig. 3 untere Kurve). Die Ergebnisse belegen die hohe Affinität und Spezifität des optischen Assays.

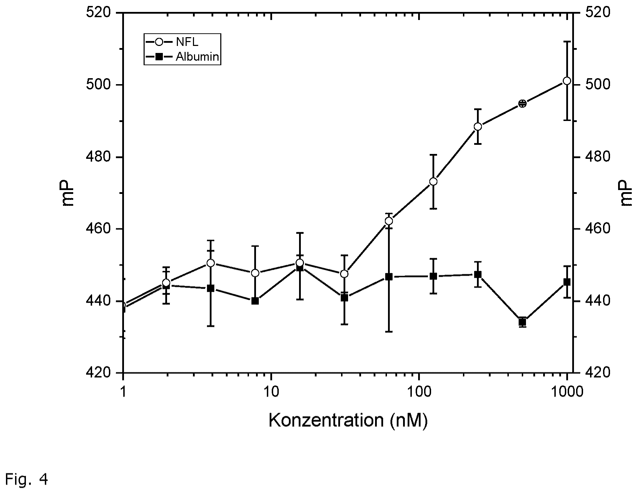

Fig. 4 zeigt eine Kalibrierkurve des optischen Assays für das erfindungsgemäße, modifizierte Aptamer_ FZJ-NFL_opt3 gemäß Beispiel 3, aufgenommen durch Fluoreszenzpolarisation als Funktion der Konzentration. Der lineare Detektionsbereich des optischen Assays startet von etwa 30 nM und reicht bis 1000 nM; der K_{D}-Wert beträgt 124,80 nM für Aptamer_ FZJ-NFL_opt3 (Fig. 4 obere Kurve). Das modifizierte Aptamer_FZJ-NFL_opt3 zeigt keine Bindungsaffinität zu Humanserumalbumin, was die hohe Spezifität der Aptamere unterstreicht (Fig. 4 untere Kurve). Die Ergebnisse belegen die hohe Affinität und Spezifität des optischen Assays.

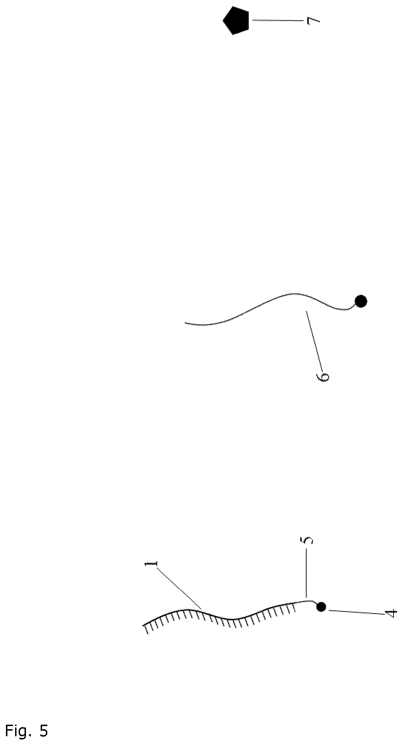

Fig. 5 zeigt eine schematische Darstellung von einsetzbaren, im Rahmen der vorliegenden Erfindung bevorzugten, chemischen Komponenten des elektrochemischen Assays. Links das Aptamer **1** inklusive an das 5'-Ende gebundene Bindungsgruppenteile **4, 5** (HS-(CH₂)₆-Aptamer), in der Mitte ein Blockierungsmolekül (Thiol-PEG 2000) **6** und rechts eine Redoxsonde ([Fe(CN)₆]^{3-/4-/}) **7.** Obwohl in dieser Figur nicht dargestellt, wird im Rahmen der vorliegenden Erfindung bevorzugt an das 3'-Ende des Aptamers ein Fluorophor gebunden, um über Änderung des Fluoreszenzverhaltens die Gegenwart des Analyten anzuzeigen (vgl. auch Fig. 1). Sofern im Folgenden im Hinblick auf die Figurenbeschreibungen von einem Aptamer gesprochen wird, ist immer die Möglichkeit eines an das 3'-Ende gebundenen Fluorophors zumindest als Alternative mit gemeint.

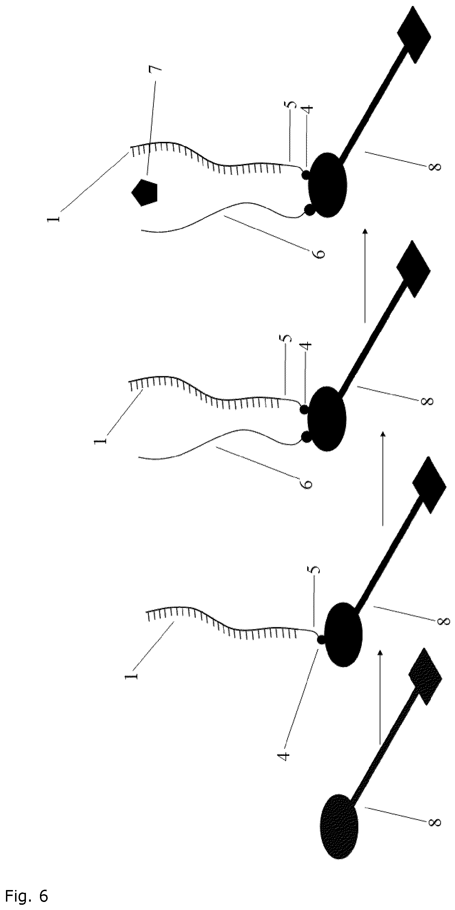

Fig. 6 illustriert schematisch die einzelnen Modifizierungsschritte für die Funktionalisierung einer Elektrode für einen elektrochemischen Assay gemäß dem erfindungsgemäßen Verfahren anhand einer Mikroelektrode auf welche zunächst eine Mikroelektrode **8** bereitgestellt wird. Dann wird eine Rezeptorschicht aufgebracht, die das Aptamer **1** umfasst. Das hier dargestellte Aptamer **1** ist ein modifiziertes Aptamer (HS-(CH₂)₆-Aptamer) **1, 4, 5** und umfasst neben dem Aptamer **1** an sich noch Bindungsgruppenteile **4** und **5.** Im nächsten Schritt ist gezeigt, wie Blockierungsmoleküle (zum Beispiel Thiol-PEG 2000) **6** auf der Elektrodenoberfläche immobilisiert werden. Schließlich ist noch gezeigt, wie Redoxsonden **7** aufgebracht werden. Mit den so hergestellten Elektroden/Assays kann anschließend die Detektion von NFL erfolgen.

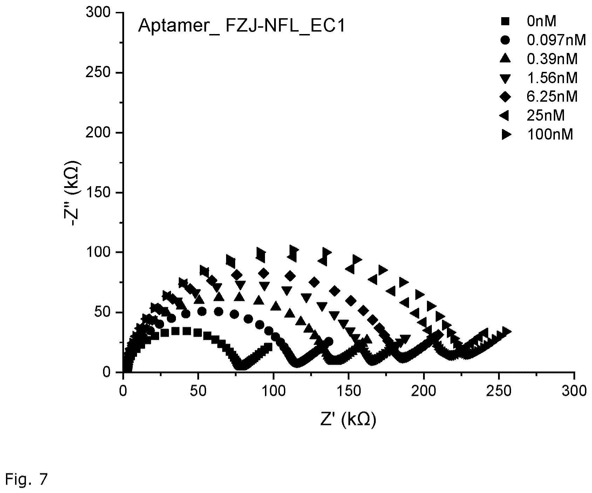

Fig. 7 zeigt in einem Nyquist-Diagramm elektrochemische Impedanzspektren aufgenommen mit einem elektrochemischen Assay basierend auf Aptamer_FZJ-NFL_EC1 gemäß Beispiel 1 für unterschiedliche NFL-Konzentrationen. Als Puffer wurde Testpuffer 1 verwendet (50 mM Tris-Acetat Puffer (50 mM tris(Hydroxymethyl) Aminomethan und Essigsäure zu pH 8.2, 5 mM [Fe(CN)₆]^{3-/4-}). Auf der Abszisse ist der Realteil und auf der Ordinate der Imaginärteil der Impedanz aufgetragen. Mit steigender NFL-Konzentration, siehe Legende, nimmt der Ladungstransferwiderstand (Minimum an der rechten Seite des Halbkreises) aufgrund der Bindung von NFL an die Aptamer-bedeckte Elektrodenoberfläche zu.

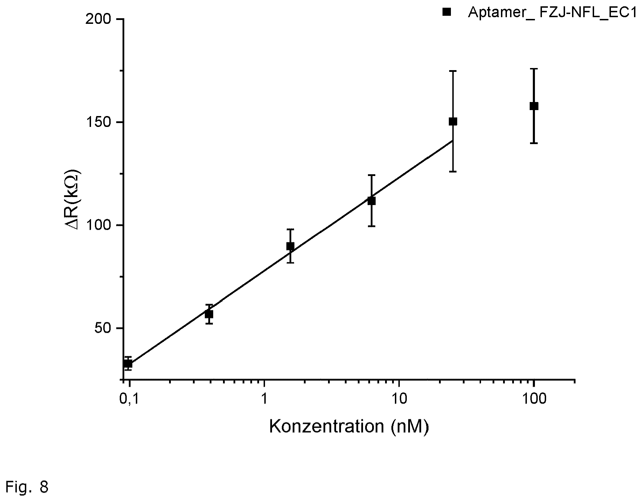

Fig. 8 zeigt die Kalibrierkurve zu Fig. 7 aufgenommen mit dem elektrochemischen Assay basierend auf Aptamer_ FZJ-NFL_EC1. Der K_{D}-Wert des Aptamers beträgt 0,34 nM. Als Signal ΔR wurde die Änderung des Ladungstransferwiderstands (Minimum an der rechten Seite des Halbkreises in Fig. 7) bei Änderung der NFL-Konzentration verwendet.

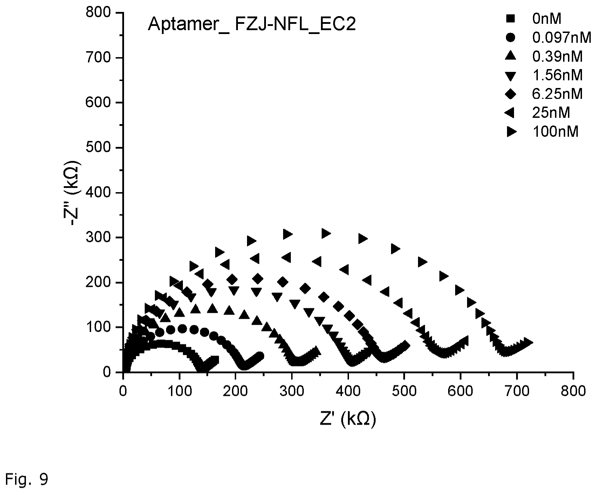

Fig. 9 zeigt in einem Nyquist-Diagramm die elektrochemischen Impedanzspektren aufgenommen mit einem elektrochemischen Assay basierend auf Aptamer_ FZJ-NFL_EC2 gemäß Beispiel 1 für unterschiedliche NFL-Konzentrationen. Als Puffer wurde Testpuffer 1 verwendet (50 mM Tris-Acetat Puffer (50 mM tris(Hydroxymethyl) Aminomethan und Essigsäure zu pH 8.2, 5 mM [Fe(CN)₆]^{3-/4-}). Auf der Abszisse ist der Realteil und auf der Ordinate der Imaginärteil der Impedanz aufgetragen. Mit steigender NFL-Konzentration, siehe Legende, nimmt der Ladungstransferwiderstand (Minimum an der rechten Seite des Halbkreises) aufgrund der Bindung von NFL an die Aptamer bedeckte Elektrodenoberfläche zu.

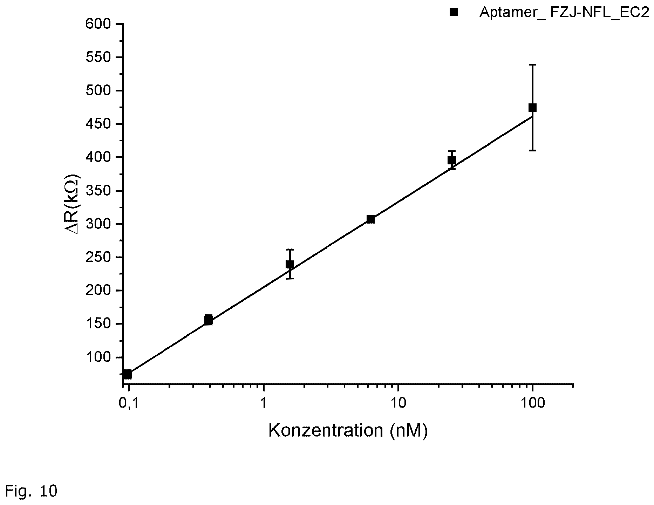

Fig. 10 zeigt die Kalibrierkurve zu Fig. 9 aufgenommen mit dem elektrochemischen Assay basierend auf Aptamer_ FZJ-NFL_EC2. Der K_{D}-Wert des Aptamers beträgt 0,41 nM. Als Signal ΔR wurde die Änderung des Ladungstransferwiderstands (Minimum an der rechten Seite des Halbkreises in Fig. 9) bei Änderung der NFL-Konzentration verwendet.

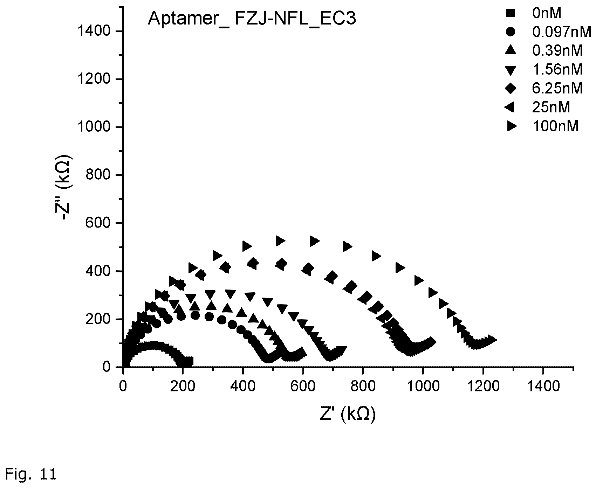

Fig. 11 zeigt in einem Nyquist-Diagramm die elektrochemischen Impedanzspektren aufgenommen mit einem elektrochemischen Assay basierend auf Aptamer_ FZJ-NFL_EC3 gemäß Beispiel 1 für unterschiedliche NFL-Konzentrationen. Als Puffer wurde Testpuffer 1 verwendet (50 mM Tris-Acetat Puffer (50 mM tris(Hydroxymethyl) Aminomethan und Essigsäure zu pH 8.2, 5 mM [Fe(CN)₆]^{3-/4-}). Auf der Abszisse ist der Realteil und auf der Ordinate der Imaginärteil der Impedanz aufgetragen. Mit steigender NFL-Konzentration, siehe Legende, nimmt der Ladungstransferwiderstand (Minimum an der rechten Seite des Halbkreises) aufgrund der Bindung von NFL an die Aptamer bedeckte Elektrodenoberfläche zu.

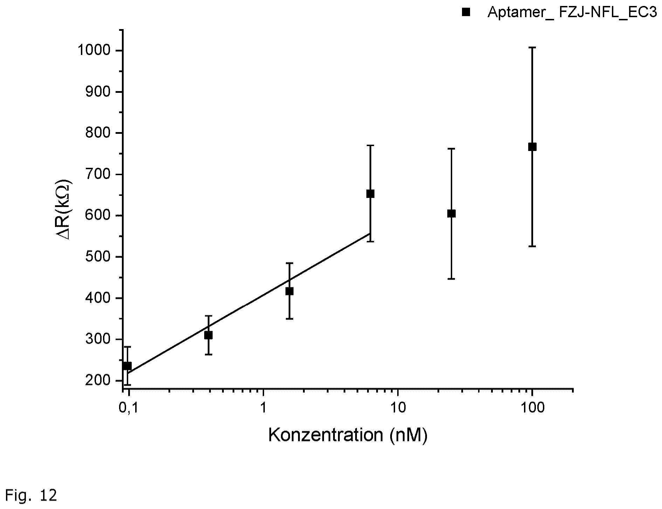

Fig. 12 zeigt die Kalibrierkurve zu Fig. 11 aufgenommen mit dem elektrochemischen Assay basierend auf Aptamer_ FZJ-NFL_EC3. Der K_{D}-Wert des Aptamers beträgt 0,18 nM. Als Signal ΔR wurde die Änderung des Ladungstransferwiderstands (Minimum an der rechten Seite des Halbkreises in Fig. 11) bei Änderung der NFL-Konzentration verwendet.

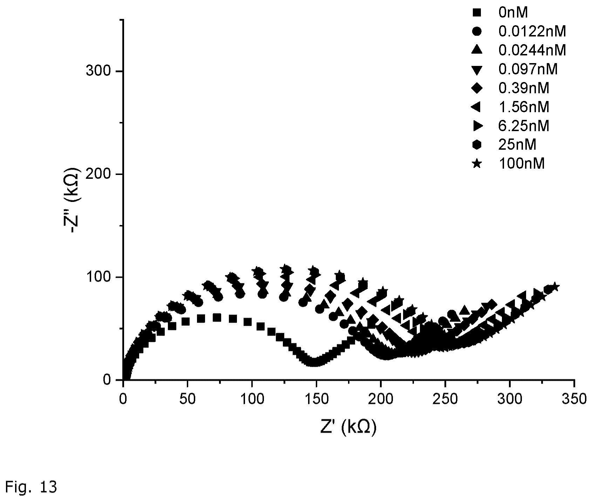

Fig. 13 zeigt in einem Nyquist-Diagramm die elektrochemischen Impedanzspektren aufgenommen mit einem elektrochemischen Assay basierend auf Aptamer_ FZJ-NFL_EC1 für unterschiedliche NFL-Konzentrationen in Phosphat-Puffer mit hoher Salzkonzentration und einem erweiterten Konzentrationsbereich. Als Puffer wurde Testpuffer 2 verwendet (10m M PBS, pH 7.4, 137 mM NaCl, 2.7 mM KCl, 5 mM [Fe(CN)₆]^{3-/4-}), Auf der Abszisse ist der Realteil und auf der Ordinate der Imaginärteil der Impedanz aufgetragen. Mit steigender NFL-Konzentration, siehe Legende, nimmt der Ladungstransferwiderstand (Minimum an der rechten Seite des Halbkreises) aufgrund der Bindung von NFL an die Aptamer bedeckte Elektrodenoberfläche zu.

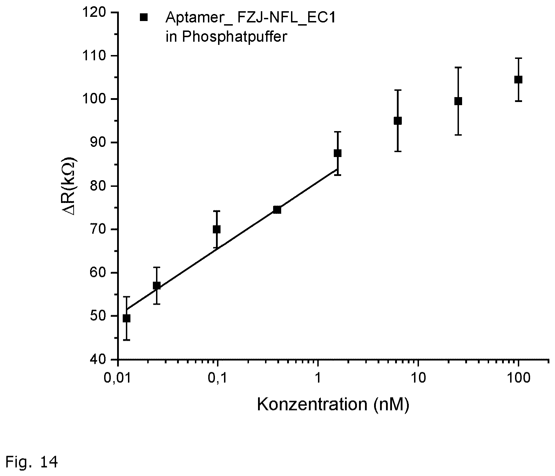

Fig. 14 zeigt die Kalibrierkurve zu Fig. 13 aufgenommen mit dem elektrochemischen Assay basierend auf Aptamer_ FZJ-NFL_EC1. Der K_{D}-Wert des Aptamers in Phosphat-Puffer mit hoher Salzkonzentration beträgt 0,23 nM. Als Signal ΔR wurde die Änderung des Ladungstransferwiderstands (Minimum an der rechten Seite des Halbkreises in Fig. 13) bei Änderung der NFL-Konzentration verwendet.

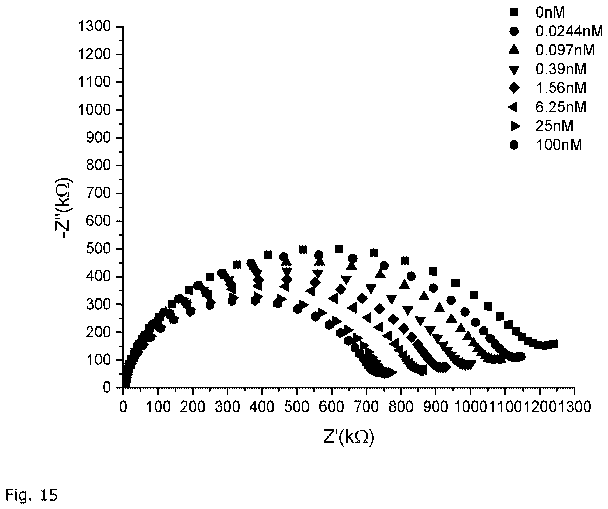

Fig. 15 zeigt in einem Nyquist-Diagramm die elektrochemischen Impedanzspektren aufgenommen mit einem elektrochemischen Assay basierend auf Aptamer_ FZJ-NFL_EC1 für unterschiedliche NFL-Konzentrationen. Als Puffer wurde humanes Serum verwendet (10fach verdünnt mit Testpuffer 2). Auf der Abszisse ist der Realteil und auf der Ordinate der Imaginärteil der Impedanz aufgetragen. Mit steigender NFL-Konzentration, siehe Legende, nimmt der Ladungstransferwiderstand (Minimum an der rechten Seite des Halbkreises) aufgrund der Bindung von NFL an die Aptamer bedeckte Elektrodenoberfläche ab.

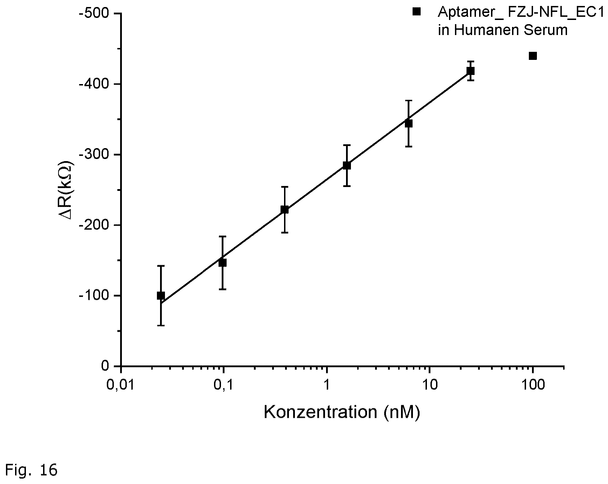

Fig. 16 zeigt die Kalibrierkurve zu Fig. 15 aufgenommen mit dem elektrochemischen Assay basierend auf Aptamer_ FZJ-NFL_EC1 in künstlichem Blut. Der K_{D}-Wert des Aptamers in künstlichem Blut beträgt 0,48 nM. Als Signal ΔR wurde die Änderung des Ladungstransferwiderstands (Minimum an der rechten Seite des Halbkreises in Fig. 15) bei Änderung der NFL-Konzentration verwendet.

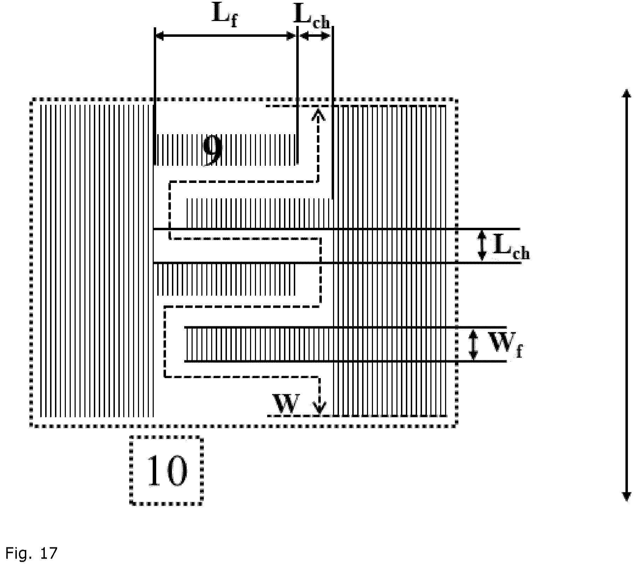

Fig.17 zeigt schematisch ein interdigitierendes Elektrodenpaar (dies bedeutet in der vorliegenden Erfindung ein Paar von Elektroden, mit ineinandergreifenden und gegenüberliegenden Elektrodenfingern) eines erfindungsgemäß einsetzbaren Transistors, wobei eine Elektrode als Source-Elektrode **12** und die andere als Drain-Elektrode **13** fungiert (die Anordnung links/rechts ist willkürlich dargestellt). Die Elektroden besitzen Finger **9,** durch die sich die Breite der Elektrodenkanals vergrößert. Auf dem Elektrodenpaar befindet sich das leitfähige Polymer **11,** das den Kanal des OECTs bildet **10.**

Die Source- und Drain-Elektroden bestehen aus interdigitierenden Elektroden, bei denen eine Anzahl an Elektrodenfingern die Breite **W** des OECT-Kanals erhöht. **L_{f}** repräsentiert Länge jedes einzelnen Elektrodenfingers. **W_{f}** definiert die Breite der Elektrodenfinger und **L_{ch}** gibt die Länge des OECT-Kanals an (Abstand zwischen Source- und Drain-Elektrode). Die Anzahl an Finger ist im hier gezeigten Beispiel zwei, kann aber im Rahmen der vorliegenden Erfindung erhöht werden, um den Verstärkungsfaktor zu erhöhen. Die Anzahl der Finger wird gegen den Flächenbedarf des OECTs optimiert. **L_{f}** beträgt zum Beispiel 25 Mikrometer und **W** 30 Mikrometer. **W_{f}** entspricht 5 Mikrometer, was der Kanallänge **L_{ch}** entspricht. Die Gate-Elektrode besitzt zum Beispiel einen Durchmesser von 0,5 Millimeter und der Abstand zum Kanal beträgt zum Beispiel 50 Mikrometer.

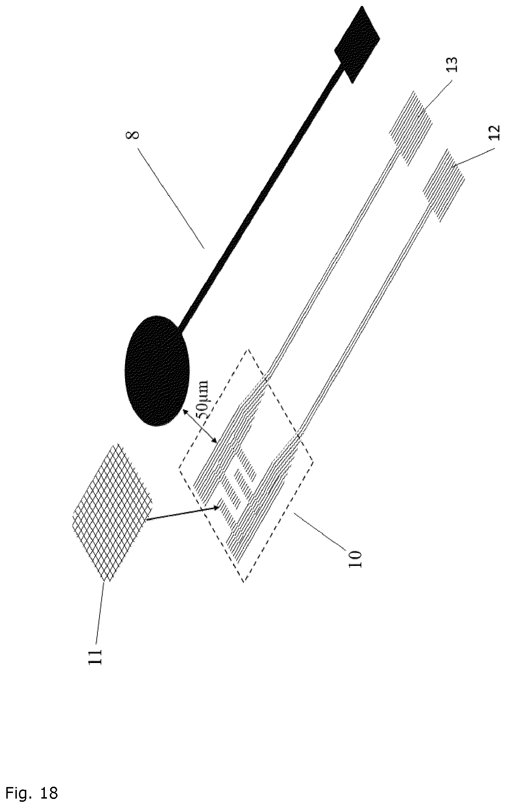

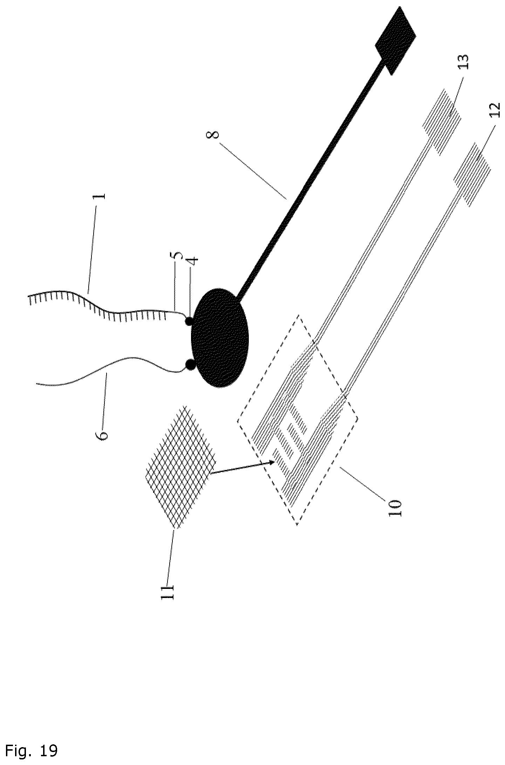

Fig.18 zeigt schematisch ein interdigitierendes Elektrodenpaar gemäß Fig. 17 mit dazugehörigen Zuleitungen und Kontaktflächen sowie dem leitfähigen Polymer **11,** das sich auf dem Kanal **10** befindet. Zusätzlich wird die Gate-Elektrode **8** des erfindungsgemäß einsetzbaren Transistors gezeigt, die sich mit einem Abstand von 50 µm in der Nähe des interdigitierenden Elektrodenpaars **12, 13** mit **10** und **11** befindet. Die Gate-Elektrode wird in dieser Figur seitlich angeordnet dargestellt. Die vorliegende Erfindung ist aber nicht darauf beschränkt; es ist zum Beispiel genausogut möglich, die Gate-Elektrode auf der gegenüberliegenden Seite bzw. ober- oder unterhalb anzuordnen (was bei der Implementierung gegebenenfalls aufwändiger sein kann). Genauso ist der Abstand der Gate-Elektrode nicht auf den angegebenen Wert beschränkt. Es sei darauf hingewiesen, dass zuerst die Metallelektroden hergestellt werden und anschließend das Polymer darauf abgeschieden wird; um dies zu verdeutlichen, wird in Fig.18 und 19 Polymer **11** mit einem Pfeil versehen dargestellt, was bedeutet, dass er später abgeschieden wird, nicht dass Polymer **11** auf den Elektroden **12, 13** liegt.

Fig.19 illustriert die Anordnung gemäß Figur 18, wobei zusätzlich auf der Gate-Elektrode **8** ein modifiziertes Aptamer, bestehend aus **1, 4** und **5,** und ein Blockierungsmolekül (zum Beispiel Thiol-PEG 2000) **6** vorhanden sind.

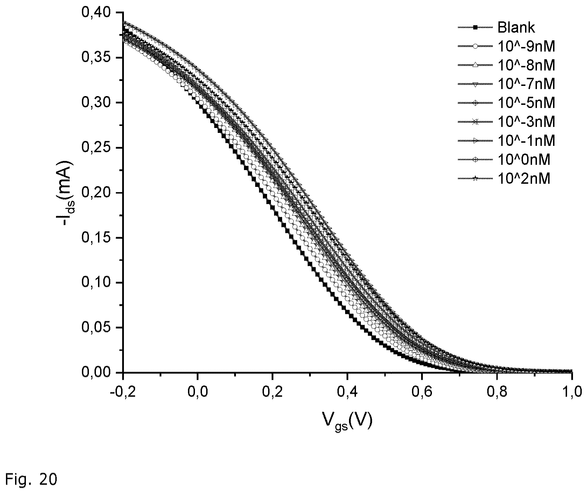

Fig. 20 zeigt die Transferkurven eines OECTs gemäß Beispiel 2 und entsprechend Fig. 19 basierend auf Aptamer_ FZJ-NFL_EC1 in PBS-Puffer (PBS = phosphatgepufferte Salzlösung (phosphate buffered saline)) mit unterschiedlichen Konzentrationen an NFL. Auf der Abszisse ist die Spannung zwischen Source und Gate-Elektrode aufgetragen und auf der Ordinate der Strom, der zwischen Source und Drain Elektrode fließt. Die Messungen wurden mit einer Source-Drain Spannung von -0,1 V und einer Gate-Spannung von -0,2 V bis 1,0 V aufgenommen. Die Schrittweite betrug 10 mV und die Spannungsvorschubrate 137 mV/s.

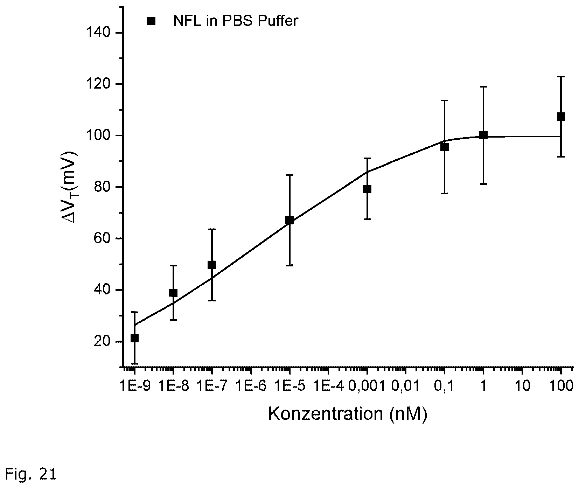

Fig. 21 zeigt die Kalibrierkurve zu Fig. 20 aufgenommen mit dem Assay basierend auf einem OECT in Kombination mit Aptamer_ FZJ-NFL_EC1 in PBS-Puffer mit unterschiedlichen Konzentrationen an NFL. Als Signal ΔV_{T} wurde die Änderung der Der K_{D}-Wert des Aptamers beträgt 0,003 nM.

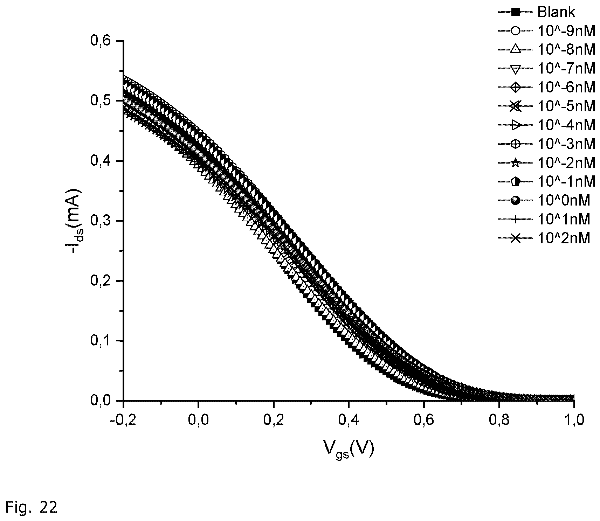

Fig. 22 zeigt die Transferkurven eines OECTs gemäß Beispiel 2 und entsprechend Fig. 19 basierend auf Aptamer_ FZJ-NFL_EC1 in humanem Serum mit unterschiedlichen Konzentrationen an NFL. Auf der Abszisse ist die Spannung zwischen Source und Gate-Elektrode aufgetragen und auf der Ordinate der Strom, der zwischen Source und Drain Elektrode fließt. Die Messungen wurden mit einer Source-Drain Spannung von -0,1 V und einer Gate-Spannung von -0,2 V bis 1,0 V aufgenommen. Die. Schrittweite betrug 10 mV und die Spannungsvorschubrate 137 mV/s.

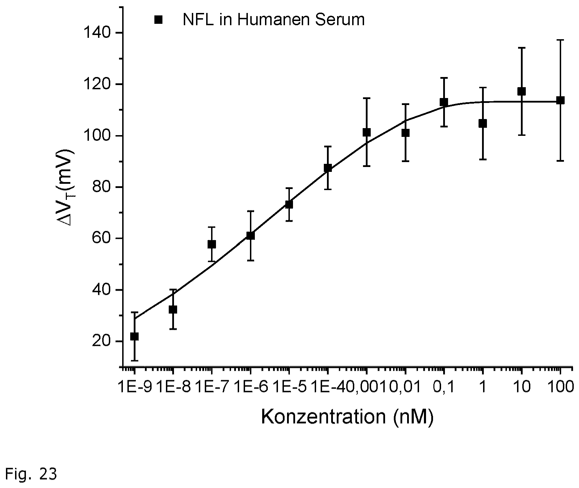

Fig. 23 zeigt die Kalibrierkurve zu Fig. 22 aufgenommen mit dem Assay basierend auf einem OECT in Kombination mit Aptamer_ FZJ-NFL_EC1 in humanen Serum mit 10fach verdünnt mit Testpuffer 2 mit unterschiedlichen Konzentrationen an NFL. Als Signal ΔV_{T} wurde die Änderung der Threshold-Spannung (=Schwellenspannung bezeichnet den Wert, bei welcher der Strom gerade beginnt zu fließen) bei Änderung der NFL-Konzentration verwendet. Der K_{D}-Wert des Aptamers beträgt 0,003 nM.

Fig. 24 illustriert die zweidimensionale Struktur des erfindungsgemäßen Aptamers FZJ-NFL_1, welches drei Stem-Loop Strukturen aufweisen kann. Die Nummerierung startet vom 5' Ende und setzt sich zum 3' Ende fort. Entsprechend sind besonders Nukleotide 1 bis 10 (GCATACTTGC), Nukleotide 15 bis 23 (CCTAACAGG), Nukleotide 31 bis 43(CAAGGTACGATTG) für die Funktionalität des Aptamers entscheidend.

Fig. 25 illustriert die zweidimensionale Struktur des erfindungsgemäßen Aptamers FZJ-NFL_2. Die Stem-Loop Strukturen umfassen die Nukleotide 14 bis 40 (GCCTTTATTTCGTATGTCGTCGAAGGT), wobei die Nukleotide 18 bis 22 (TTATT) keine Basenpaarung aufweisen.

Fig. 26 illustriert die zweidimensionale Struktur des erfindungsgemäßen Aptamers FZJ-NFL_3. Die Struktur kann zwei Stem-Loop Strukturen ausbilden, welche Nukleotide 5 bis 12 (GCCCGCGC) sowie Nukleotide 19 bis 35 (CGTAGACACACCTAACG) umfassen.

### Bezugszeichenliste:

In den Figuren bedeuten gleiche Bezugszeichen gleiche Materialien, Stoffe etc.
- 1: Aptamer
- 2: Fluorophor
- 3: Analyt (Neurofilament Light - NFL)
- 4: Bindungsgruppenteil (zum Beispiel HS-S-Gruppe (Disulfid-Brücke)
- 5: Bindungsgruppenteil (zum Beispiel -(CH₂)₆-Kette)
- 6: Blockierungsmolekül (zum Beispiel Thiol-PEG 2000)
- 7: Redoxsonde (zum Beispiel [Fe(CN)₆]^{3-/4-/})
- 8: Mikroelektrode (Gate-Elektrode)
- 9: Elektrodenfinger
- 10: OECT-Kanal (Kanal des organischen elektrochemischen Transistors mit interdigitierenden Elektrodenfingern)
- 11: leitfähiges Polymer
- 12: Mikroelektrode (Source-Elektrode)
- 13: Mikroelektrode (Drain-Elektrode)
- W: Breite des OECT-Kanals
- W_{f}: Breite eines Elektrodenfingers
- L_{f}: Länge eines Elektrodenfingers
- L_{ch}: Länge des OECT-Kanals

Die vorliegende Erfindung wird nun unter Heranziehung der folgenden nicht-limitierenden Beispiele näher erläutert. Die folgenden nicht-limitierenden Beispiele dienen dazu die darin ausgeführten Ausgestaltungen darzulegen. Dem Fachmann ist bekannt, dass Variationen dieser Beispiele im Rahmen der vorliegenden Erfindung möglich sind.

### Beispiele:

Die Funktionalität der folgenden drei Ausführungsbeispiele wurde anhand des Nachweises von Humanem Neurofilament Light Polypeptid (NFL) belegt, das in E. coli exprimiert wurde (Biozol Biovender, Molekulargewicht 62.5 kDa).

### Beispiel 1: Elektrochemischer Assay

Dieses Ausführungsbeispiel beschreibt einen Sensor, der auf einem elektrochemischen Transducer beruht und eine quantifizierbare Korrelation zwischen Messsignal und NFL in der Probe ermöglicht. Der Sensor beruht auf Metallelektroden, die als Einzelelektroden oder als Multielektrodenfelder vorliegen können. In diesem Ausführungsbeispiel wurden Goldelektroden verwendet. Für die Herstellung des Sensors wurden die Elektroden gereinigt durch jeweils 5 Minuten Spülung in Aceton und Isopropanol, gefolgt von einer Spülung mit hochreinem entionisierten Wasser (aus einem Milli-Q^{®}-System (18,2 Megaohm spezifischer Widerstand)) und Trocknung im Stickstofffluss. Die elektrochemische Reinigung der Elektroden erfolgte durch zyklische Voltametrie (CV) in 0,1 M NaOH in einem Potentialbereich von -1,35 V bis - 0,35 V während 10 Scans bei 2 V s-1 und anschließendem Scannen in 0,05 M H₂SO₄ in einem Potentialbereich von 0 V bis 1,5 V während 20 Scans bei 1 V s-1. Die elektrochemische Oberflächenfläche (ESA) wurde mittels CV in 0,05 M H₂SO₄ in einem Potentialbereich von 0 V bis 1,5 V bei 0,1 V s-1 bestimmt. Es sei hier angemerkt, dass die genaue Durchführung der Reinigung stark abhängig von dem verwendeten Elektrodenmaterial ist und beispielsweise Plasmareinigungsschritte oder andere enthalten kann, wobei hier nur die in diesem Beispiel ausgeführte Reinigung wiedergegeben ist.

Nach der Reinigung (Aktivierung) der Elektrode wurden die Aptamere über Bindungsgruppen (im vorliegenden Fall Thiolbindung) an die Oberflächen der Elektroden gebunden.

In diesem Ausführungsbeispiel wurden die Aptamere mit einer am 5' Ende der ssDNA als Bindungsgruppe modifiziert, wodurch sich folgende Sequenzen ergeben:
Aptamer_ FZJ-NFL_EC1, HS-(CH₂)₆- GCA TAC TTG CGA GTC CTA ACA GGT ATT GGT CAA GGT ACG ATT G
Aptamer_ FZJ-NFLEC2, HS-(CH₂)₆- TCC CTC CAT CTA AGC CTT TAT TTC GTA TGT CGT CGA AGG TGT G
Aptamer_ FZJ-NFL_EC3, HS-(CH₂)₆- CAT AGC CCG CGC AAT CTA CGT AGA CAC ACC TAA CGA AAC CAA C

Diese modifizierten Aptamere wurden in diesem Beispiel mit einer Konzentration von jeweils 0,05 µM verwendet. Alle Aptamere wurden mit 10 mM Tris-(2-carboxyethyl)-Phosphinhydrochlorid (TCEP)-Lösung während einer Stunde bei Raumtemperatur inkubiert, um die Disulfid-Schutzbindung zu spalten und die Immobilisierung auf den Elektroden über eine Thiol-Gold-selbstorganisierende Monoschicht zu ermöglichen. Die Aptamerlösungen wurden in 50mM Tris-Acetat-Puffer (50mM Tris(hydroxymethyl) Aminomethan 1M NaCl, 1 mM MgCl₂, pH 8.20) gemischt. Die Elektroden wurden dann für 16 Stunden mit der jeweiligen Aptamerlösung unter Ausschluss von Licht inkubiert. Die Aptamer-modifizierten Elektroden wurde zunächst mit Tris-Acetat-Puffer gespült und anschließend mit entionisiertem Wasser (Milli-Q^{®}-Wasser), um nicht spezifisch adsorbierte Moleküle zu entfernen.

Die Elektroden wurde für 1 Stunde mit einer 5 mg/ml monofunktionellen Methoxy-Polyethylenglykolthiol-Lösung (1.5mg/mL Thiol-PEG 2000) inkubiert. Das PEG diente als Blockierungsmolekül, um Biofouling durch andere vorhandene Moleküle in den Proben zu vermeiden (unspezifische Bindung von Matrixkomponenten). Der Aptasensor wurde mit 50 mM Tris-Acetat-Puffer (pH 8.2) und Milli-Q^{®}-Wasser gewaschen, um überschüssige, nicht spezifisch adsorbierte PEG-Moleküle zu entfernen.

Elektrochemische Impedanzspektroskopie-Messungen (EIS) wurden durchgeführt, um die Detektion von NFL in den Proben durch die Aptamersensoren zu bestimmen. Hierfür wurde der Sensor für 30 Minuten in 50 mM Tris-Acetat Puffer (50 mM tris(Hydroxymethyl) Aminomethan und Essigsäure zu pH 8.2, 5 mM [Fe(CN)₆]^{3-/4-}; Testpuffer 1) oder in 10m M PBS (pH 7.4, 137 mM NaCl, 2.7 mM KCl, 5 mM [Fe(CN)₆]^{3-/4-}; Testpuffer 2) oder in oder humanen Serum 10fach verdünnt mit Testpuffer 2 inkubiert wobei die Lösungen unterschiedlichen aber definierte Konzentrationen an NFL enthielten.

Die EIS-Messungen wurden mit einem Autolab-Potentiostaten (Metrohm) mit einem Dreielektrodensystem durchgeführt. Dabei wurde ein Platindraht als Gegenelektrode (CE), eine Ag/AgCl-Elektrode als Referenzelektrode (RE) und die Aptamer-modifizierten Elektroden als Arbeitselektroden (WE) eingesetzt. Die EIS-Messungen wurden bei einem Potenzial von 0,22 V durchgeführt, mit einer Wechselspannungsamplitude von 0,01 V und einem Frequenzbereich von 0,1-10 kHz. Die statistische Analyse der Detektion mit den verschiedenen Elektroden und den verschiedenen Aptameren wurde mittels statistischer Analysesoftware durchgeführt und ausgewertet.

Die Messergebnisse sind in Fig. 7 bis 16 dargestellt.

Für die Implementierung des elektrochemischen Sensors wurde die Elektrode, die die Rezeptorschicht enthielt, einer Lösung ausgesetzt, die den Analyten in unterschiedlichen, aber bekannten Konzentrationen enthielt (Konzentrationen siehe Legende, gekennzeichnet durch unterschiedliche Symbole). Anschließend wurden die zugehörigen Impedanzspektren gemessen, siehe Fig. 7, 9, 11, (es wurde jeweils ein anderer Aptamer pro Figur verwendet, siehe Legende). Bei den Puffern handelte es sich um Tris-Puffer (Testpuffer 1). Aus den erhaltenen Nyquist-Diagrammen kann der Ladungstransferwiderstand (Minimum an der rechten Seite des Halbkreises) ermittelt werden, welcher durch die Bindung von NFL an die Aptamer-bedeckte Elektrodenoberfläche steigt. Dieser Anstieg des Ladungstransferwiderstandes wird durch den erschwerten Ladungstransfer zwischen Elektrode und Redoxsonde (Ferri/Ferrocyanid) in der Lösung hervorgerufen. Der Ladungstransfer wird durch sterische und elektrostatische Abschirmungseffekte der an die Aptamerrezeptoren gebundenen NFL-Moleküle hervorgerufen, sodass die Redoxsonden die Elektrode nur schwer erreichen. Dies ist durch die Zunahme des Durchmessers des Halbkreises im Impedanzspektrum zu erkennen.

Anschließend wurde aus den ermittelten Werten des Ladungstransferwiderstandes die Änderung des Ladungstransferwiderstandes berechnet, die man erhält, wenn eine bestimmte Konzentration an NFL dem Sensor ausgesetzt wird (ΔR=R_{ohne NFL}-R_{mit NFL}). Diese Änderung des Ladungstransferwiderstandes wurde dann gegen die jeweilige Konzentration an NFL aufgetragen, wodurch man die Kalibrierungskurve erhielt (Fig. 8, 10, 12). Diese Kurve gibt an, welche Änderung des Ladungstransferwiderstandes man bei einer bestimmten Konzentration messen kann. Wird eine Lösung unbekannter NFL-Konzentration dem Sensor ausgesetzt, so kann man über die Änderung des Ladungstransferwiderstandes die Konzentration an NFL bestimmen.

Für die weitere Implementierung des elektrochemischen Sensors wurde die Elektrode, die die Rezeptorschicht enthielt, einer Lösung ausgesetzt, die den Analyten in unterschiedlichen, aber bekannten Konzentrationen enthielt (Konzentrationen siehe Legende, gekennzeichnet durch unterschiedliche Symbole). Anschließend wurden die Impedanzspektren gemessen, siehe Fig. 13, (Aptamer_FZJ-NFL_EC1). Der Puffer bestand aus PDS-Puffer (Testpuffer 2). In der Abbildung ist ein Anstieg des Ladungstransferwiderstandes zu beobachten, der durch den erschwerten Ladungstransfer zwischen Elektrode und Redoxsonde (Ferri/Ferrocyanid) in der Lösung hervorgerufen wird. Der Ladungstransfer wird durch sterische und elektrostatische Abschirmungseffekte des an die Aptamerrezeptoren gebundenen NFL-Moleküle hervorgerufen, sodass die Redoxsonden die Elektrode nur schwer erreichen. Dies ist durch die Zunahme des Durchmessers des Halbkreises im Impedanzspektrum zu erkennen.

Anschließend wurden aus den ermittelten Werten des Ladungstransferwiderstandes wiederum die Änderung des Ladungstransferwiderstandes berechnet. Diese Änderung des Ladungstransferwiderstandes wurde dann gegen die jeweilige Konzentration an NFL aufgetragen, wodurch man die Kalibrierungskurve erhält (Fig. 14). Diese Kurve gibt an, welche Änderung des Ladungstransferwiderstandes man bei einer bestimmten Konzentration im Testpuffer 2 messen kann.

Für die Implementierung des elektrochemischen Sensors in realen Proben wurde die Elektrode, die die Rezeptorschicht enthielt, einer Lösung aus verdünntem humanen Serum ausgesetzt, das den Analyten in unterschiedlichen, aber bekannten Konzentrationen enthielt (Konzentrationen siehe Legende, gekennzeichnet durch unterschiedliche Symbole). Anschließend wurden die Impedanzspektren gemessen, siehe Fig. 15 (Aptamer_FZJ-NFL_EC1). Aus den erhaltenen Nyquist-Diagrammen konnte wiederum der Ladungstransferwiderstand ermittelt werden, welcher in diesem Fall durch die Bindung von NFL an die Aptamer-bedeckte Elektrodenoberfläche sank. Diese Reduzierung des Ladungstransferwiderstands wird durch den leichteren Ladungstransfer zwischen Elektrode und Redoxsonde (Ferri/Ferrocyanid) in der Lösung hervorgerufen. Der leichtere Ladungstransfer wird durch eine Veränderung des NFL in humanen Serum hervorgerufen, die zu einer positiveren Nettoladung führt, aber die Affinität zum Aptamer nicht beeinträchtigt. Diese positive Ladungsverschiebung reduziert die elektrostatische Abstoßung zwischen dem Rezeptorfilm und dem negativ geladenen Redoxsonden und erleichtern somit den Ladungstransfer. Die Reduzierung des Ladungstransferwiderstands ist durch die Abnahme des Durchmessers des Halbkreises im Impedanzspektrum zu erkennen.

Anschließend wurde aus den ermittelten Werten des Ladungstransferwiderstandes die Änderung des Ladungstransferwiderstandes berechnet, die man erhält, wenn eine bestimmte Konzentration an NFL dem Sensor ausgesetzt wird (ΔR=R_{ohne NFL}-R_{mit NFL}). Da der Ladungstransferwiderstand mit zunehmender Konzentration an NFL abnimmt, sind die ΔR-Werte negativ. Diese Änderung des Ladungstransferwiderstandes wurde dann gegen die jeweilige Konzentration an NFL aufgetragen, wodurch man die Kalibrierungskurve erhielt (Fig. 16). Diese Kurve gibt an, welch eine Änderung des Ladungstransferwiderstandes man bei einer bestimmten Konzentration messen kann.

### Beispiel 2: Assay unter Verwendung eines organischen elektrochemischen Transistors

Dieses Ausführungsbeispiel beschreibt einen Sensor, der auf einem organischen elektrochemischen Transistor (OECT) oder Feldern (Arrays) von OECTs als Transducer beruht und eine quantifizierbare Korrelation zwischen Messsignal und NFL in der Probe ermöglicht. Der hier verwendete organisch elektrochemische Transistor beinhaltete drei Elektroden, wobei eine als Source-Elektrode, eine als Drain-Elektrode und eine als Gate-Elektrode verwendet wurde (vgl. auch Fig. 17). Zwischen Source- und Drain-Elektrode befindet sich ein Kanal, der aus einem leitfähigen Polymer besteht, Fig. 18.

Die hier verwendeten Elektroden bestanden aus Gold und bei dem leitfähigen Polymer handelte es sich um Poly-3,4-ethylendioxythiophen/Poly(styrolsulfonsäure) (PEDOT/PSS). Der OECT wurde in dieser Ausführung als Verstärker betrieben, um sehr kleine Detektionsgrenzen für den Nachweis von NFL zu ermöglichen. Hierfür wurden die Gate-Elektroden mit dem Aptamer_ FZJ-NFL_EC1 modifiziert, siehe oben in Beispiel 1 zum elektrochemischen Assay. Zudem kann der Aptamer auf oder im Kanalmaterial gebunden werden. Bei der Immobilisierung der Aptamere auf der Gate-Elektroden wurden diese zudem für 1 Stunde in Blockierungsmolekülen einer 1,5 mg/ml monofunktionellen Methoxy-Polyethylenglykolthiol-Lösung (1,5 mg/mL Thiol-PEG 2000) inkubiert. Der Nachweis von NFL wurde einerseits in PBS-Puffer (vgl. auch Fig. 20 und Fig. 21 - 10 mM PBS pH 7,4, 137 mM NaCl, 2.7 mM NaCl)) und andererseits in humanen Serum 10fach verdünnt mit Testpuffer 2 (Fig. 22 und Fig. 23) vorgenommen. Die Inkubationszeit betrug 30 Minuten.

Der Kanal bestand aus einer Mischung von Poly(3,4-Ethylendioxythiophen)/Polystyrensulfonat (PEDOT:PSS) mit 20 Gew.-% D-Sorbitol, 1 Vol.-% (3-Glycidyloxypropyl)trimethoxysilan (GOPS), und 0.1 Vol.-% 4-Dodecylbenzenesulfonsäure (DBSA). Die Gewichts- und Volumenverhältnisse sind relativ zum Gewicht und Volumen von PEDOT:PSS. Diese Mischung wurde mit 1000 rpm für 10 Sekunden und 4000 rpm für 30 Sekunden auf die Chipoberfläche aufgeschleudert. Anschließend wurde der Polymerfilm auf 120°C erhitzt und für 20 Minuten getempert. Fig. 17 (siehe oben) zeigt Details zur Kanalgeometrie des in diesem Beispiel eingesetzten Transistors.

Die Messergebnisse sind in Fig. 20, 21, 22 und 23 dargestellt.

Fig. 20 zeigt die Transferkurven eines OECTs gemäß Fig. 19 basierend auf Aptamer_ FZJ-NFL_EC1 in PBS-Puffer (PBS = phosphatgepufferte Salzlösung (phosphate buffered saline)) mit unterschiedlichen, aber bekannten Konzentrationen an NFL. Hierfür wurde des Aptamer FZJ-NFL_EC1 und Thiol-PEG 2000 auf der Gate-Elektrode immobilisiert. Auf der Abszisse von Fig. 20 ist die Spannung zwischen Source und Gate-Elektrode des OECTs aufgetragen und auf der Ordinate der Strom, der zwischen Source und Drain Elektrode fließt. Die Messungen wurden mit einer konstanten Source-Drain Spannung von -0,1 V und einer Gate-Spannung von -0,2 V bis 1,0 V aufgenommen. Durch das Anlegen der Source / Drain Spannung fließt ein definierter Source / Drain Strom (I_{ds}) durch dem PEDOT:PSS-Kanal, der eine Funktion des Kanalwiderstandes ist. Der Kanalwiderstand und somit I_{ds} ist aber auch von der Spannung zwischen Gate und Source Elektrode (V_{gs}) abhängig. Steigt V_{gs} an, wandern positiv geladene Ionen aus dem Elektrolyten in den leitfähigen Polymer des OECT-Kanals, kompensieren die negativ geladenen Sulfonatgruppen des PSS und reduzieren die Dotierung des Polymers, wodurch der Kanalwiderstand ansteigt und I_{ds} fällt. Wird die Gate-Elektrode mit der Rezeptorschicht modifiziert und einer NFL-haltigen Lösung ausgesetzt, so induziert die Bindung dieser Moleküle eine Variation der Gate-Source-Spannung V_{gs}, was zu einer Änderung von I_{ds} führt. Somit korrespondiert die Änderung der Transferkurve (I_{ds} vs. V_{gs}) mit der NFL-Bedeckung an der Aptamer-modifizierten Gate-Elektrode und folglich auch der NFL-Konzentration in der Lösung.

Aus der Transferkurve kann die Schwellspannung (V_{T}) von V_{gs} bestimmt werden, bei der es zu einem nennenswerten Anstieg von I_{ds} kommt (bei dieser Spannung wird der Kanal leitend, hier bestimmt durch den Schnittpunkt der Tangente (mit maximalen Steigung) mit der Abszisse). Trägt man die Änderung an der Schwellspannung (ΔV_{T}), die man bei Änderung der NFL-Konzentration erhält, gegen die NFL-Konzentration auf, so erhält man die Kalibrierkurve Fig. 21 korrespondierend zu Fig. 20. Diese Kurve gibt an, welche Änderung des Signals ΔV_{T} man bei einer bestimmten Konzentration messen kann. Wird eine Lösung unbekannter NFL-Konzentration dem Sensor ausgesetzt, so kann man über die Änderung von V_{T} die Konzentration an NFL bestimmen.

Analog zu den Messungen in PBS-Puffer wurden die gleichen Experimente mit OECTs in verdünnten humanen Serumproben durchgeführt, bei denen die Gate-Elektrode mit FZJ-NFL_EC1 und Thiol-PEG 2000 modifiziert wurde. Fig. 22 zeigt die zugehörigen Transferkurven eines OECTs bei unterschiedlichen NFL-Konzentrationen in humanen Serum 10fach verdünnt mit Testpuffer 2. Auch hier führt die zunehmende Immobilisierung des NFL an die Gate-Elektrode zu einer Verschiebung der Transferkurve zu höheren V_{gs} Werten, wodurch auch V_{T} zunimmt. Wird diese konzentrationsabhängige Änderung der Schwellspannung (ΔV_{T}) gegen die NFL-Konzentration im verdünnten humanen Serum aufgetragen, so erhält man die Kalibrierkurve, mit deren Hilfe die NFL-Konzentration einer unbekannten Lösung bestimmt werden kann.

### Beispiel 3: Optischer Assay

In diesem Ausführungsbeispiel wird ein optischer Assay beschrieben, der auf Fluoreszenzpolarisationsmessungen beruht. Auch dieser Sensor erlaubte eine quantifizierbare Korrelation zwischen Messsignal und NFL in der Probe.

Hierfür wurden die Aptamere mit cy5 Fluoreszenzmolekülen am 5' Ende modifiziert. Da dieser Assay in der Lösung ohne Immobilisierung des Aptamers erfolgt, wurde auf eine Bindungsgruppe verzichtet.

Die in diesem Beispiel verwendeten modifizierten Aptamere waren die folgenden:
Aptamer_ FZJ-NFL_opt1, cy5- GCA TAC TTG CGA GTC CTA ACA GGT ATT GGT CAA GGT ACG ATT G
Aptamer FZJ-NFL_opt2, cy5- TCC CTC CAT CTA AGC CTT TAT TTC GTA TGT CGT CGA AGG TGT G
Aptamer_ FZJ-NFL_opt3,- cy5- CAT AGC CCG CGC AAT CTA CGT AGA CAC ACC TAA CGA AAC CAA C

Für den optischen Assay wurde NFL in 50 mM Tris-Acetat-Puffer (pH 8.2) gelöst. Corning^{®} 384 Multiwell Platten wurden mit Protein-Blockierpuffer (Pierce^{™} Protein-Free (TBS) Blocking Buffer, Thermal Fisher Scientific) für 1 Stunde beschichtet. Anschließend wurden die Blockierpuffer entfernt und die Multiwell Platten in einem Inertgasstrom getrocknet.

Die Cy5-modifizierten Aptamere (10 nM) wurden mit NFL-haltiger humane Serumlösung ansteigender Konzentration gemischt und für 30 Minuten inkubiert. Anschließend wurde das Fluoreszenzpolarisationssignal gemessen. Die Anregungswellenlänge wurde auf 650 nm gesetzt und die Emission bei einer Wellenlänge bei 670 nm gemessen.

Die Messergebnisse sind in Fig. 2, 3 und 4 dargestellt.

In diesen Experimenten wurde ein optischer Assay realisiert, der auf Fluoreszenzpolarisationsmessungen beruht. Dieser Sensor erlaubte eine quantifizierbare Korrelation zwischen Messsignal (Fluoreszenzpolarisation) und NFL in der Probe. Bei diesen Messungen wurde die Änderung der Fluoreszenzpolarisation gemessen, die durch die Bindung eines fluoreszenzmarkierten Aptamers mit seinem Targetmolekül hervorgerufen wird. Hierfür wurde die Aptamerlösung mit linear polarisiertem Licht durchleuchtet und der Fluorophor angeregt. In der Regel ist das emittierte Fluoreszenzlicht ebenfalls linear polarisiert, wird jedoch durch die Rotationsdiffusion der gelösten Aptamermoleküle beeinflusst. Um die Polarisierung P des Fluoreszenzlichtes zu bestimmen, wird die Intensität (I_{II}) der parallelen Strahlung gemessen, wenn Polarisator (vor Probe) und Analysator (hinter der Probe) parallel zueinander stehen. Zudem wird die die Intensität (I_{⊥}) gemessen, wenn Polarisator und Analysator senkrecht zueinander stehen. Die Polarisation berechnet sich aus der Differenz von paralleler Intensität zu senkrechter Intensität gemessen, geteilt durch die Summer beider P=( I_{II}-I_{⊥})/( I_{II}+I_{⊥}). Für die Untersuchungen wurden die Aptamere mit cy5 Fluoreszenzmolekülen am 5' Ende modifiziert, da cy5 eine geeignete Fluoreszenzlebensdauer für Fluoreszenzpolarisationsuntersuchungen hat. Für Fig.2 wurde eine FZJ-NFL_opt1 Lösung mit NFL-Lösungen unterschiedlicher, aber bekannter NFL-Konzentrationen inkubiert. Mit zunehmender Assoziation zwischen Aptamer und NFL kommt es zu steigenden Werten für die Fluoreszenzpolarisation, da die Masse des Proteins sehr viel größer ist als die des Aptamers und somit die Rotationsdiffusion des NFL-Aptamerkomplexes abnimmt, was dazu führt, dass sich die Ausrichtung der Fluorophore wenig ändern und die Polarisation des Anregungslichtes stärker im Emissionslicht erhalten bleibt.

Aus der Abhängigkeit der Polarisation des Fluoreszenzlichtes von der NFL-Konzentration kann eine Kalibierkurve bestimmt werden. Wenn anschließend die Polarisation des Fluoreszenzlichtes einer Lösung unbekannter Konzentration gemessen wird, dann kann mithilfe der gefitteten Funktion die Konzentration bestimmt werden. Der Fit kann, wie hier geschehen, beispielsweise über eine Hill-Gleichung erfolgen.

Neben NFL wurde auch vergleichbarer Bindungstest mit Albumin durchgeführt, dass in großen Konzentrationen im Blut vorliegt. Im Gegensatz zum NFL ändert sich die Fluoreszenzpolarisation auch mit steigenden Konzentrationen von Albumin nicht, was belegt, dass der Aptamer nicht an Albumin bindet und somit eine hohe Spezifität für NFL aufweist.

Vergleichbar zu den Experimenten, die mit dem Aptamer FZJ-NFL_opt1 für Fig. 2 aufgenommen wurden, sind Experimente mit dem Aptamer FZJ-NFL_opt2 durchgeführt worden und in Fig. 3 dargestellt, ebenso wie mit Aptamer FZJ-NFL_opt3, dessen Ergebnisse in Fig. 4 gezeigt werden. Alle drei Aptamere können NFL in Lösung binden und weisen eine hohe Selektivität gegenüber Albumin auf.

## Patentansprüche

1. Aptamer, das an Neurofilament Light bindet und das ausgewählt ist aus der Gruppe bestehend aus
a) einem Aptamer umfassend, oder bestehend aus, eine/einer Nukleinsäuresequenz, die ausgewählt ist aus der Gruppe bestehend aus SEQ ID NO: 1, 2 und 3;
b) einem Aptamer, dessen Nukleinsäuresequenz eine Übereinstimmung von mindestens 50%, 60%, 65%, 70%, 75%, 80%, 85%, 90%, 95% oder 98% mit der Nukleinsäuresequenz eines Aptamers aus a) aufweist; oder
c) einem Aptamer, bei dem gegenüber einem Aptamer aus a) bei der Sequenz mit der SEQ ID NO: 1, oder der SEQ ID NO: 2, oder der SEQ ID NO: 3 bis zu 19 Nukleotide, entfernt, substituiert und/oder erweitert sind.

2. Aptamer nach Anspruch 1, **dadurch gekennzeichnet, dass**
- das Aptamer durch Bindung an ein Sondenmolekül modifiziert ist, insbesondere über eine kovalente Bindung, wobei das Sondenmolekül bevorzugt ausgewählt ist aus der Gruppe bestehend aus Fluorophoren, Nanomaterialien, Enzymen, Redoxmolekülen oder Mischungen oder Kombinationen davon;
oder
- das eingesetzte Aptamer durch Bindung an ein Sondenmolekül modifiziert ist, insbesondere über eine kovalente Bindung, wobei das Sondenmolekül bevorzugt ausgewählt ist aus der Gruppe bestehend aus Fluorophoren, Nanomaterialien, Enzymen, Redoxmolekülen oder Mischungen oder Kombinationen davon, und das eingesetzte Aptamer zusätzlich an seinem anderen Ende durch Bindung an eine Bindungsgruppe modifiziert ist, wobei die Bindungsgruppe bevorzugt ausgewählt ist aus der Gruppe bestehend aus Monothiolen, Multithiolen, Disulfiden R-S-S-R', R-NH₂ Gruppen, R-CS₂ Gruppen, R-CN Gruppen, wobei R und R'= beliebige lineare aliphatische oder aromatische Moleküle mit einer Kettenlänge kürzer 100 nm und R von R' verschieden sein kann, Methyl-Carbodithioat-Estern, kovalenter Bindung und Mischungen davon.

3. Aptamersonde zur Detektion von Neurofilament Light umfassend ein oder mehrere verschiedene Aptamere gemäß Anspruch 1 oder 2 und ein Markierungsmittel.

4. Biosensor umfassend
- ein an Neurofilament Light bindendes Aptamer gemäß Anspruch 1 oder 2,
wobei das Aptamer entweder
a) frei in einer Probenlösung vorliegt, oder
b) an einer festen Phase immobilisiert vorliegt.

5. Biosensor nach Anspruch 4, **dadurch gekennzeichnet, dass**
in Variante a) das eingesetzte Aptamer durch Bindung an ein Sondenmolekül modifiziert ist, insbesondere über eine kovalente Bindung, wobei das Sondenmolekül bevorzugt ausgewählt ist aus der Gruppe bestehend aus Fluorophoren, Nanomaterialien, Enzymen, Redoxmolekülen oder Mischungen oder Kombinationen davon;
oder
in Variante b) das eingesetzte Aptamer durch Bindung an ein Sondenmolekül modifiziert ist, insbesondere über eine kovalente Bindung, wobei das Sondenmolekül bevorzugt ausgewählt ist aus der Gruppe bestehend aus Fluorophoren, Nanomaterialien, Enzymen, Redoxmolekülen oder Mischungen oder Kombinationen davon, und das eingesetzte Aptamer zusätzlich an seinem anderen Ende durch Bindung an eine Bindungsgruppe modifiziert ist, wobei die Bindungsgruppe bevorzugt ausgewählt ist aus der Gruppe bestehend aus Monothiolen, Multithiolen, Disulfiden R-S-S-R', R-NH₂ Gruppen, R-CS₂ Gruppen, R-CN Gruppen, wobei R und R'= beliebige lineare aliphatische oder aromatische Moleküle mit einer Kettenlänge kürzer 100 nm und R von R' verschieden sein kann, Methyl-Carbodithioat-Estern, kovalenter Bindung und Mischungen davon.

6. Biosensor nach einem der Ansprüche 4 oder 5, **dadurch gekennzeichnet, dass** in Variante b) zusätzlich Moleküle, welche die unspezifische Bindung von Komponenten der Probe unterdrücken an einer festen Phase immobilisiert sind.

7. Verfahren zur Bestimmung von Neurofilament Light in Körperflüssigkeiten und/oder Stuhl umfassend die Schritte:
a) Bereitstellen eines an Neurofilament Light bindenden Aptamers, insbesondere gemäß Anspruch 1 oder Anspruch 2;
b) Inkontaktbringen des Aptamers mit einer Probe der Körperflüssigkeit und/oder des Stuhls;
c) Detektieren der Bindung von Neurofilament Light an das Aptamer.

8. Verfahren nach Anspruch 7, **dadurch gekennzeichnet, dass** das Aptamer in Form eines Biosensors gemäß einem der Ansprüche 4 bis 6 bereitgestellt wird.

9. Verfahren nach Anspruch 7 oder 8, **dadurch gekennzeichnet, dass** die Detektion mittels
- optischer Methoden, bevorzugt Fluoreszenzspektroskopie, oder
- elektrischer Methoden, bevorzugt Messung von Spannungsänderungen, erfolgt.

10. Verfahren zur Herstellung der Biosensoren gemäß einem der Ansprüche 4 bis 6 umfassend die Schritte
- Bereitstellen eines an Neurofilament Light bindenden Aptamers;
- optional Modifizieren des Aptamers;
- optional Bereitstellen einer Elektrode, einer Transistorelektrode, einer Mikrowaage, oder einem anderem Signalwandler;
- Immobilisieren des Aptamers auf der Elektrode, der Mikrowaage oder dem anderen Signalwandler;
- optional Immobilisieren von Blockierungsmolekülen auf der der Elektrode, der Mikrowaage oder dem anderen Signalwandler.

11. Verwendung der Aptamere gemäß einem der Ansprüche 1 oder 2, der Aptamersonden nach Anspruch 3, der Biosensoren gemäß einem der Ansprüche 4 bis 6, des Verfahrens gemäß einem der Ansprüche 7 bis 9,
- zum Nachweis von Neurofilament Light in Proben;
- zur Anreicherung, Abtrennung und/oder Isolierung von Neurofilament Light aus Proben;
- für potentiell präventive und dezentrale Testung, insbesondere breiter Bevölkerungsgruppen.

12. Verwendung der Aptamere gemäß einem der Ansprüche 1 oder 2 oder der Aptamersonden nach Anspruch 3 als Therapeutika, als Affinitätstags, als Reagenzien, zum Markieren von Systemen, die Neurofilament Light enthalten, für oder in Biosensoren, zur Qualitätskontrolle.

13. Verwendung eines Sequenzmotivs umfassend die Nukleotide 23 bis 32 aus SEQ ID NO: 1 zur Bindung eines dieses Sequenzmotiv enthaltenden Aptamers an Neurofilament light.

14. Verwendung eines Sequenzmotivs mit der Nukleotiden GTATTGGTCA zur Bindung eines dieses Sequenzmotiv enthaltenden Aptamers an Neurofilament light.

15. Oligonukleotid umfassend die Nukleotidsequenz GTATTGGTCA oder bestehend aus der Nukleotidsequenz GTATTGGTCA.
